# EUROPEAN PATENT SPECIFICATION

(11) **EP 3 607 090 B1**
(45) Date of publication and mention of the grant of the patent: **02.07.2025**
(21) Application number: 18781399.3
(22) Date of filing: 06.04.2018
(51) Int. Cl.: C12Q 1/68, G01N 33/52

(54) **HIGH PRECISION MICROPURIFICATION SYSTEM AND METHODS OF USE**
HOCHPRÄZISIONSMIKROREINIGUNGSSYSTEM UND VERFAHREN ZUR VERWENDUNG
SYSTÈME DE MICROPURIFICATION DE PRÉCISION ÉLEVÉE ET PROCÉDÉS D'UTILISATION

(30) Priority: 07.04.2017 US 201762482833 P; 14.03.2018 US 201862642872 P
(43) Date of publication of application: 12.02.2020
(73) Proprietor: AVONEAUX MEDICAL INSTITUTE LLC, Clarkston, MI 48348 (US); LIFEBRIDGE INNOVATIONS LLC, Baltimore, MD 21215 (US)
(72) Inventor: Emmert-Buck, Michael R., Clarkston, MI 48348 (US); Tangrea, Michael Anthony, Baltimore, MD 21215 (US)
(74) Representative: Danner, Stefan
(86) International application number: PCT/US2018/026469
(87) International publication number: WO 2018/187697

(56) References cited:
- US-A- 4 659 655
- US-A- 5 225 325
- US-A- 5 395 541
- US-A1- 2007 128 719
- US-A1- 2008 064 098
- US-A1- 2009 186 065
- SHINJI SAKAI ET AL: "Cell surface coating with hydrogel sheath through on-cell surface enzymatic cross-linking", FRONT. BIOENG. BIOTECHNOL., 30 March 2016 (2016-03-30), pages 1 - 2, XP055673369, DOI: 10.3389/conf.FBIOE.2016.01.00707
- MARY K. COWMAN ET AL: "The Content and Size of Hyaluronan in Biological Fluids and Tissues", FRONTIERS IN IMMUNOLOGY, vol. 6, 2 June 2015 (2015-06-02), CH, XP055672307, ISSN: 1664-3224, DOI: 10.3389/fimmu.2015.00261
- KARA A. DAVIS ET AL: "Coatings on mammalian cells: interfacing cells with their environment", JOURNAL OF BIOLOGICAL ENGINEERING, vol. 13, no. 1, 17 January 2019 (2019-01-17), XP055672287, DOI: 10.1186/s13036-018-0131-6

## Description

### FIELD OF THE INVENTION

Histology methods are provided that target and allow isolation of specific types of cells or structures using high-precision processing techniques as specified in the claims.

### BACKGROUND

Biological samples such as histological tissue sections are inherently complex, containing numerous cell types (e.g., nerve cells, vasculature cells, epithelium cells, fibroblasts, etc.) as well as extracellular structures (e.g., extracellular matrix, etc.) that enable their specialized physiological functions. Quantitative molecular analysis of cells from histological slides is useful in developing a deeper understanding of fundamental pathophysiology and in generating effective clinical interventions [1-15]. While this complexity enables specialized functions of each tissue type, it can confound scientific or clinical analysis of individual types of cells within the tissue microenvironment.

For pathological diseases such as cancer, isolating tumor cells is further complicated by tumor heterogeneity, as a histology sample may comprise normal cells and cancerous cells, as well as regions of hyperplasia or neoplasia. Additionally, a tumor focus may contain multiple clones arising during tumor development and progression, with each clone having a unique expression or mutation profile. Thus, a histological tissue section may include a heterogeneous population of normal cells, a heterogeneous population of tumor cells and other structures present within the tissue and/or tumor microenvironment that complicate analysis.

In the early 1990s, Shibata demonstrated that cells coated with ink by mechanical process were protected from UV radiation (see, Shibata et al., Am. J. Path. (1992) vol. 141, no. 3, pp. 539-543). However, this technique was not cell-specific, typically covering groups of cells and protecting any cell covered by ink regardless of its particular cell type.

Several other strategies were developed in the 1990s and early 2000s to isolate cells from heterogeneous tissue sections, including manual scraping, micromanipulators, laser capture microdissection (LCM), and mesodissection [16-32]. Manual scraping involves removal of cells (e.g., using a razor, scraper, or other suitable tool) in a specific region of the tissue slide, and is typically performed under a microscope. While this technique is low cost, spatial resolution is low and non-target cell types may be captured along with the target cells. Micromanipulators have also been used to manually remove cells, as part of a process that also is typically performed under a microscope.

Microdissection techniques have also been used to isolate regions of cells from a tissue or histology slide. Such techniques may utilize immunohistochemistry (IHC)-based methods to identify target cells in order to achieve improved yield and precision. For example, immuno-laser capture microdissection has been used as a guide to the dissection process (see, Emmert-Buck et al., Science (1996), vol. 274: 8, pp. 998-1001). Other immunodissection methods also have been developed, including computer-based stain recognition software programs, expression microlabeling (xML), expression microdissection (xMD) (see, Hanson et al., Nature Protocols (2011) vol. 6: 4, pp. 457-467), AutoScanXT Software, etc. While such laser microdissection methods maintain spatial resolution suitable for isolating cells in heterogeneous environments, such techniques remain costly, are labor and time intensive, are performed manually typically under a microscope, and are subject to operator error. Mesodissection platforms offer yet another platform to isolate cells, by utilizing microfluidics tools to extract cells from particular regions of formalin-fixed paraffin-embedded tissue. However, mesodissection also is typically performed manually and prone to operator error, and is subject to contamination. US 4,659,655 discloses a method for the separation of target cells from a population of living cells by an enzyme-mediated shielding of the target cells from the lethal action of a drug applied to the cell population. Sakai and coworkers (Sakei et al.

(2016) Front. Bioeng. Biotechnol. Conference Abstract, doi: 10.3389) describe a hydrogel cell coating through on-cell surface enzymatic cross-linking of cell surface. However, the method does not involve the forming of any protective barrier. Davis and coworkers (Davis, K.A. et al. (2019) J. Biol. Engineer.13, 5) review methods for the coating of mammalian cells and the impact of the intended application on the design of the coating method.

While laser-based dissection technology has greatly advanced modern biology and molecular pathology [8, 18, 33-46], in spite of these successes, laser dissection instruments do not offer an easy way to isolate target cells, and fail to meet the needs of modern molecular pathology and personalized medicine. Moreover, laser dissection devices are expensive to purchase and maintain, are time- and labor-intensive to use, and are incapable of efficiently dissecting samples at a cellular level of precision.

With the advent of personalized medicine and in view of the heterogeneity of different types of tumors, techniques to accurately isolate specific types of tumor cells from individual patients for further analysis and with minimal human intervention are needed. Additionally, inexpensive options are needed for investigators or clinicians who lack access to commercial dissection instruments, and such options should allow procurement of cells by cell type in a precise manner.

Accordingly, while the aforementioned techniques are used, or have been used, for microdissection, they are subject to a variety of drawbacks, are not precise and generally not optimized for high throughput techniques involving personalized medicine. As the fields of biological research, molecular pathology, and precision medicine continue to evolve and coalesce, novel techniques will be needed to isolate and study the molecular content of specific cell populations in order to better understand biology and disease processes.

### SUMMARY

A method for isolating and collecting cells at a cellular/subcellular level of precision as specified in claim 1 is provided herein. A biological sample comprising cells is labeled with a specific probe that directly carries or produces a protective barrier that is deposited onto the cells to which the probe is bound, forming a physical and/or molecular barrier that selectively covers the labeled cells and does not cover unlabeled cells. A micropurification solution is applied to the biological sample that selectively degrades or differentially processes (e.g., contains an RNase that degrades RNA) cells or cellular components not covered by the protective barrier. The labeled cells covered with the barrier are not degraded or processed by the micropurification solution, or are degraded or processed differentially, e.g., in a different manner or to a different degree that allows recovery of the labeled cells and not the unlabeled cells.

In particular, the present invention relates to a method of collecting cells comprising: contacting a histological sample affixed to a surface with a specific probe to form a bound complex comprising cells labeled with said specific probe, wherein said specific probe comprises a reactive moiety; contacting said bound complex comprising the reactive moiety with a substrate that reacts with said reactive moiety to deposit a chemical material onto the surface of said cells labeled with said specific probe, wherein said chemical material forms a barrier covering the labeled cells or subcellular structures; and applying a micropurification solution to the biological sample, wherein said micropurification solution degrades, digests, or otherwise differentially processes cells or structures not protected by the barrier; wherein: (i) said specific probe comprises an antibody, a nucleotide-based hybridization probe, an affimer, or an aptamer;c(ii) said reactive moiety comprises an enzyme, with said substrate being a substrate for said enzyme; and (iii) said substrate is a chromogenic reagent that reacts with the reactive moiety to produce an insoluble chemical material.

After the micropurification solution is added, both the nontarget cells/structures and the target cells/structures may be separately recovered. The nontarget cells are solubilized or otherwise differentially processed by the micropurification solution and may be recovered in the solution. The target cells remain on the slide due to the microprotection effect of the barrier and can be subsequently recovered by slide scraping, further chemical processing steps, or other recovery means.

Specific cells or structures can be recovered from the biological sample (e.g., from the histological slide) because the probe binds to specific target cell(s) or structures based upon their physical or molecular characteristics, e.g., cell surface antigens, nucleotide sequences, etc.). These characteristics allow target cells to be specifically labeled by a probe (e.g., an antibody, a nucleotide-based hybridization probe, an affimer, an aptamer, etc.) that produces a precipitate or other physical or chemical barrier that protects the labeled cells from a micropurification solution that degrades or otherwise processes uncovered cells. The high precision of the labeling leads to a corresponding high precision in the location of the protective barrier, and this precision allows isolation of a desired cell population with high resolution compared to prior methods. Moreover, because the method is probe-based it is not dependent upon a human operator to manually select target cells of interest.

### BRIEF DESCRIPTION OF THE DRAWINGS

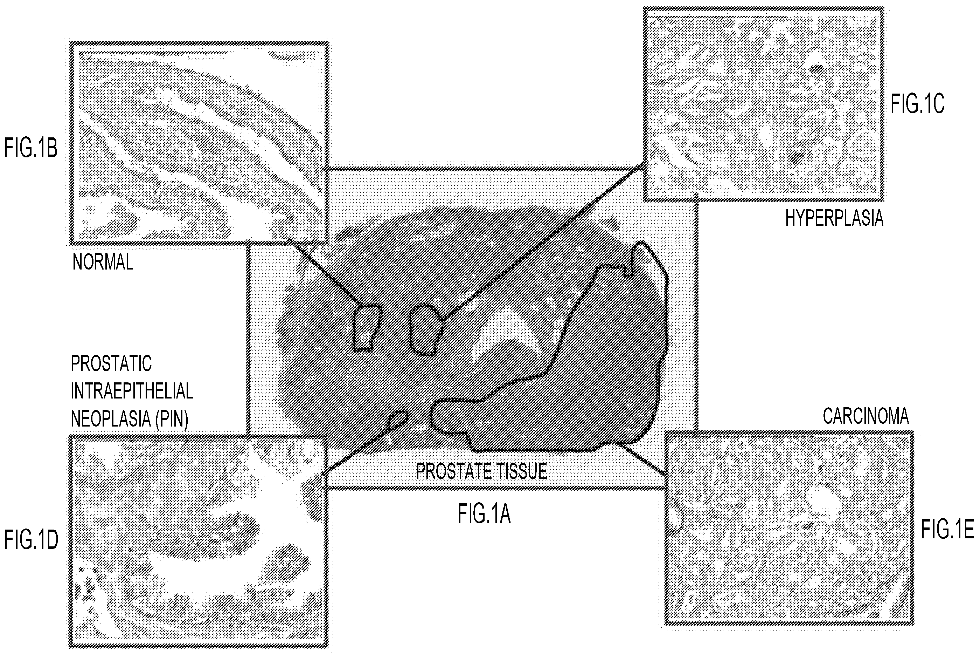

FIGs. 1A-1E show images of various prostate tissue (FIG. 1A) with differing morphologies: normal (FIG. 1B), hyperplasia (FIG. 1C), prostatic intraepithelial neoplasia (FIG. 1D), and carcinoma (FIG. 1E). As shown by this series of images, a biological sample such as prostate tissue may include cells in a variety of different states with different characteristics. Accordingly, isolating a single type of cell is often difficult.

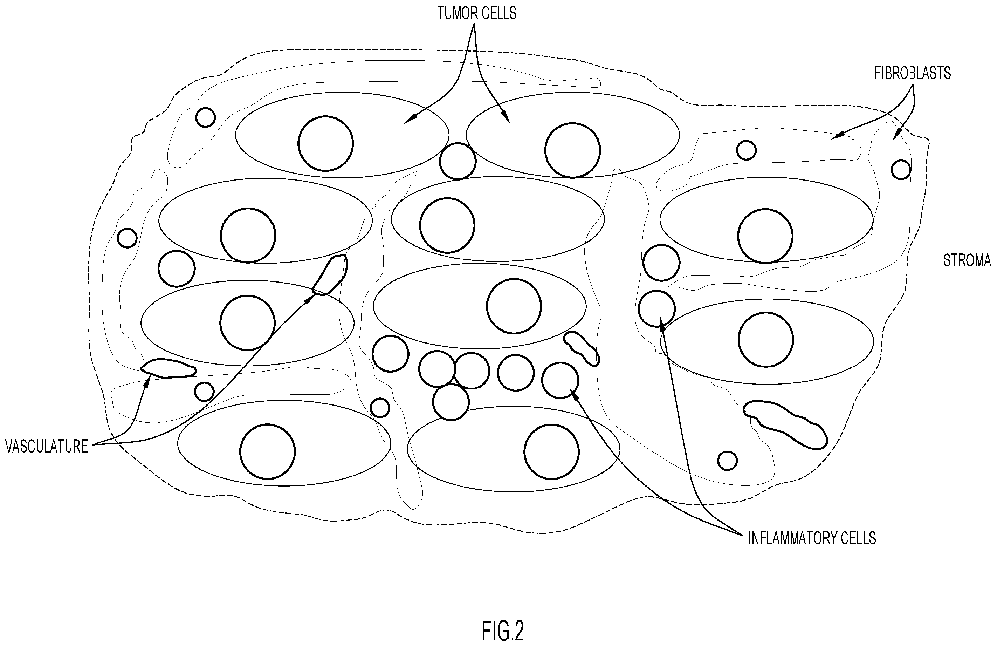

FIG. 2 shows an illustration of a biological sample, e.g., obtained by biopsy. The biological sample may contain fibroblasts (a type of normal cell), tumor cells, inflammatory cells (e.g., cells from the immune system including B and T cells), as well as vasculature and other structural components. Accordingly, not only may a type of cell, such as prostate cells, be present in various states, but also, other cell types (e.g., normal, tumor, immune, etc.) may be present in a sample.

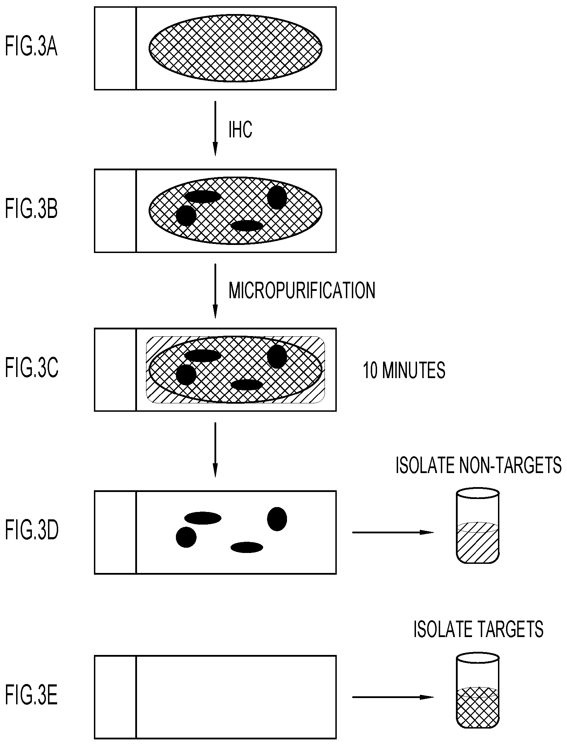

FIGs. 3A-3E show schematic drawings of various stages of the Histology Micro Protection (HMP) method provided herein and specified in the claims. The uppermost panel (FIG. 3A) shows a non-immunostained, non-labeled histological section on a glass slide. The cells and structure are represented by an oval filled with crosshatched lines. Unique physical or molecular characteristics of the target cell(s)/structures allow specific labeling by a probe, as shown in FIG. 3B, wherein target cells labeled with the probe are shown as solid black ovals or circles. The probe produces a chemical material that protects the cells (the microprotection effect) from subsequent processing as shown in FIG. 3C. The labeled cells may be incubated in a micropurification solution (shown as diagonal lines), which removes or differentially processes unlabeled cells and structures from the slide, for about 10 minutes, although other amounts of time may be suitable as well (e.g., up to 60 minutes or any amount of time in between, etc.). FIG. 3D is an illustration of the biological sample after processing. At this stage, the solubilized non-target cells are recovered by collection of the micropurification solution (shown as diagonal lines), e.g., into a test tube. As shown by FIG. 3E, the labeled target cells, which remain on the slide due to the microprotection effect, may be recovered by collecting (e.g., scraping) the cells (shown as crosshatched lines) into a test tube. Thus, FIG. 3D demonstrates negative selection with regard to the target cells, in which cells that are unlabeled are solubilized and removed from the slide, allowing a subsequent collection of the labeled cells.

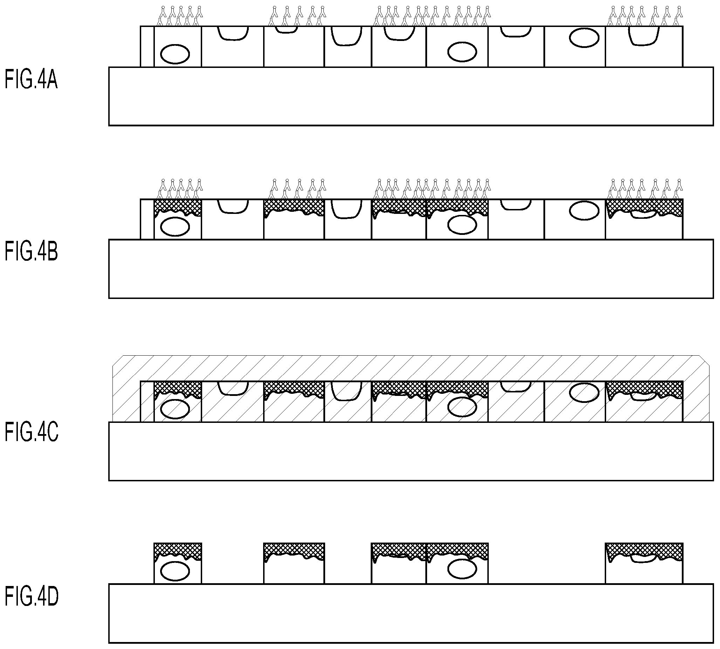

FIGs. 4A-4D show another illustration of the HMP method of the present invention. FIG. 4A shows a tissue section mounted on a glass slide. In this example, the probe may be antibody-based. A first primary antibody, having specificity for a specific antigen associated with a particular type of cell or subcellular component, is applied under conditions suitable for antibody binding to the antigen. A secondary antibody is then applied (also shown in FIG. 4A), which binds to the first primary antibody. The secondary antibody may be conjugated or may form a complex with an enzyme capable of reacting with a substrate to form an insoluble chemical material (as shown in FIG. 4B using crosshatched lines). The insoluble chemical material, or precipitate, covers the cells to which the probe is attached. FIG. 4C shows application of the micropurification solution (shown as diagonal lines) to the histology slide, and the unprotected cells (the cells not bound to a probe) are solubilized. Upon removal of the processing buffer, the labeled, covered cells remain on the slide, as shown in FIG. 4D, and may be collected by scraping or other suitable means.

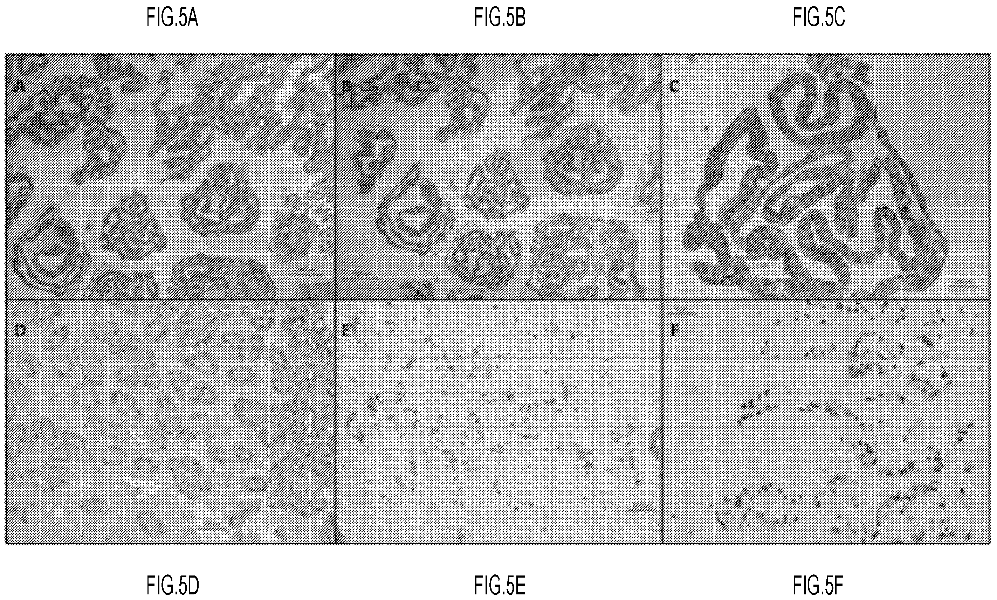

FIGs. 5A-5F show a series of microscopic images for histological sections of multiple cases of colorectal cancer before and after HMP. At FIG. 5A, colon carcinoma tissue is immunostained using cytokeratin AE1/AE3 antibodies (2X magnification). The cytokeratin AE1/AE3 antibodies bind to various cytokeratins in formalin-fixed, paraffin embedded tissue. For example, AE1 detects high molecular weight cytokeratins 10, 14, 15, and 16, and low molecular weight cytokeratin 19, while AE3 detects high molecular weight cytokeratins 1, 2, 3, 4, 5, and 6, and low molecular weight cytokeratins 7and 8. DAB is provided as a substrate, which forms an insoluble brown precipitate upon oxidation, e.g., by a peroxidase enzyme attached to or part of the probe. The target cancer cells are darkly stained while the non-target cells/structures (e.g., stroma, fibroblasts, inflammatory cells, nerves, etc.) are not darkly stained and thus are faintly visible between the stained targets. FIG. 5B shows the colon carcinoma tissue (from FIG. 5A) immunostained using cytokeratin AE1/AE3 antibodies after HMP. The stained target cells remain intact on the slide after HMP but the non-target cells were solubilized and were no longer present on the slide (2X magnification). FIG. 5C shows colon carcinoma tissue (same as FIG. 5B but at a 10X magnification) immunostained using cytokeratin AE1/AE3 antibodies after HMP.
FIG. 5D shows colon carcinoma tissue immunostained with antibodies to Ki-67 (10X magnification). For example, the MIB1 IgG1 antibody, which binds to Ki67 in formalin-fixed, paraffin embedded tissue, may be used. The target cancer nuclei are darkly stained while the non-target cells/structures (e.g., stroma, fibroblasts, inflammatory cells, nerves etc.) are not stained and thus are faintly visible between the stained targets. FIG. 5E shows colon carcinoma tissue immunostained with antibodies to Ki-67 after HMP. The stained target nuclei remain intact on the slide after HMP but the non-targets were solubilized and were no longer present on the slide (10X magnification). FIG. 5F shows colon carcinoma tissue (as in FIG. 5E but with 20X magnification) immunostained with antibodies to Ki-67 after HMP.
FIG. 6 shows an agarose gel image of DNA amplification of a 129 base pair (bp) product from HMP target cells. The data indicate that DNA is present in the recovered target cells and can be successfully amplified by PCR after the HMP process.
FIGs. 7A-7B show microscopic images of human colon cancer before HMP (FIG. 7A) and after HMP (FIG. 7B). In this example, cytokeratin was selected as an antigen, and DAB was used to form an insoluble chemical material (brown) when oxidized, e.g., by a peroxidase enzyme conjugated to or complexed with the probe.
FIGs. 8A-8B show microscopic images of human colon cancer before HMP (FIG. 8A) and after HMP (FIG. 8B). In this example, cytokeratin was selected as an antigen, and DAB was used to form an insoluble chemical material (brown) when oxidized.
FIGs. 9A-9B show microscopic images of human colon cancer before HMP (FIG. 9) and after HMP (FIG. 9B). In this example, a cluster of differentiation antigen (pan-CD) was selected as an antigen to label lymphocytes, and DAB was used to form an insoluble chemical material (brown).
FIGs. 10A-10B show microscopic images of human colon cancer before HMP (FIG. 10A) and after HMP (FIG. 10B). In this example, cytokeratin was selected as an antigen, and DAB was used to form an insoluble chemical material (brown).
FIGs. 11A-11B show microscopic images of human colon cancer before HMP (FIG. 10A) and after HMP (FIG. 10B). In this example, pan-CD was selected as an antigen, and DAB was used to form an insoluble chemical material (brown).
FIGs. 12A-12B show microscopic images of human breast cancer before HMP (FIG. 12A) and after HMP (FIG. 12B). In this example, pan-CD was selected as an antigen, and DAB was used to form an insoluble chemical material (brown).
FIGs. 13A-13B show microscopic images of human colon cancer before HMP (FIG. 13A) and after HMP (FIG. 13B). In this example, epithelium membrane antigen (EMA) was selected as an antigen, and Fast Red was used to form an insoluble chemical material.
FIGs. 14A-14B shows microscopic images of human colon cancer before HMP (FIG. 14A) and after HMP (FIG. 14B). In this example, Ki-67 was selected as an antigen, and Fast Red was used to form an insoluble chemical material.
FIGs. 15 shows a microscopic image of human colon cancer after HMP. In this example, Ki-67 was selected as an antigen, and DAB was used to form an insoluble chemical material.
FIG. 16 shows a list of samples which have been processed according to the HMP techniques provided herein. As shown in this figure, HMP was successfully used to isolate the tumor strains (see arrows), while the non-tumor cells were not detected.

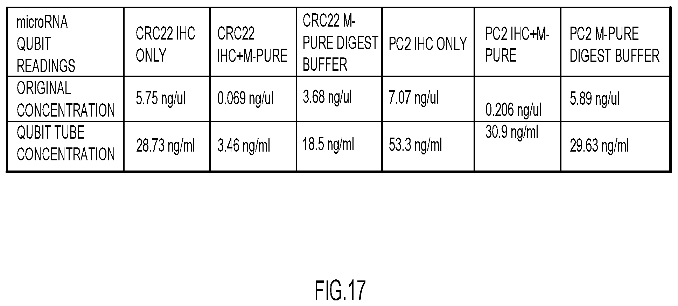

FIG. 17 shows results of an IHC procedure with cases: PC2 (x2) and CRC22 (x2) followed by micropurification, RNA extraction, and microRNA Qubit reading. The primary antibody was 1:100 cytokeratin. Conditions for the micropurification buffer were 1mg/ml proteinase K in Tris buffer with 1% SDS. The slide was scraped after IHC for RNA extraction, scraped after micropurification for RNA extraction, and the digest buffer from micropurification was collected for RNA extraction. Results, which are an average of triplicate reading, showed successful recovery of mRNA. A standard antigen retrieval procedure was used, e.g., microwave treatment using Citra buffer as described herein.

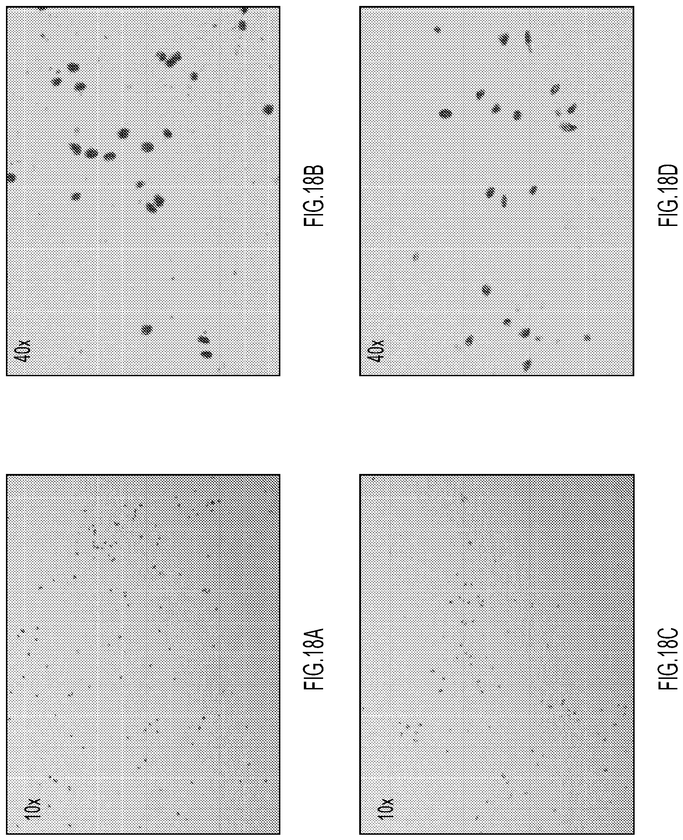

FIGs. 18A-18D show nuclei from two sets of experiments at different magnifications from human breast cancer FFPE tissue that was isolated using micropurification, and stained with a histone primary antibody and a secondary antibody labeled with alkaline phosphatase. The chromogen was NBT/BCIP.

### DETAILED DESCRIPTION

A Histology Micro Protection (HMP) method as specified in the claims is provided that allows high resolution selection of cells (e.g., tumor cells) or subcellular structures at a molecular level using a probe that produces a chemical material that covers the cell to which the probe is attached. HMP utilizes and integrates novel probe-based targeting and microprotection effects to isolate specific types of cells or structures in a complex biological sample comprising a plurality of different cell types. The isolated cells can be subsequently analyzed by any of a variety of techniques, including, e.g., expression profiling, NGS, etc.

HMP methods of the present invention provide increased resolution, higher throughput, and greater ease of use compared to current cell selection methods and technologies such as manual dissection, laser capture microdissection, or an immuno-based dissection method. For example, HMP can recover specific cells or subcellular structures from histology slides within minutes without relying on complex instrumentation involving laser dissection, slide irradiation, and/or microscopy. Using HMP, cells or subcellular structures are recovered based on their molecular characteristics. Accordingly, HMP reagents may also be provided as part of a kit (not part of the present invention) to provide cell selection capability in the absence of complex technologies. Additionally, HMP provides a high level of precision, allowing isolation of a specific type of cell with high yield, as compared to current techniques which typically rely on complex instrumentation and produce lower yields than HMP. These techniques may also be used to isolate a specific type of cell present at a low concentration within a biological sample.

In one aspect, the HMP method of the present invention is used to isolate cells on a histological slide carrying a biological sample. The histological slide may be generated according to techniques known in the art. For example, a biological sample is sectioned, formalin-fixed, and embedded in paraffin, and may undergo additional processing to expose the antigens (e.g., antigen retrieval) so that probes can bind to the antigen. The slide is then incubated with the probe, wherein the probe attaches to cells of interest based upon the cell's molecular characteristics. A moiety on the probe forms, e.g., directly or as a byproduct, a barrier that coats the cells to which the probe is attached. The labeled cells are then incubated in a micropurification solution for a period of time sufficient to solubilize, differentially process, and/or degrade cells that are not coated with the chemical material. The time required to solubilize, differentially process, and/or degrade cells may vary depending on the tissue but may be, for example, 10 minutes. The attached labeled cells may then be removed (e.g., by scraping, etc.) into another container. This process is also shown in FIGs. 3A-3E and FIGs. 4A-4D. Additional aspects of this process are provided as follows.

### Histology, Cytology, and Cell Culture Slides, Flasks, and Petri Dishes

Histology samples typically are thinly sliced, or sectioned, such that the individual sections may be stacked (vertically) to reconstruct the 3-dimensional structure of the biological sample. Accordingly, the histological slide may include extracellular targets, targets on the surface of a cell, or targets within the cell (e.g., the cytoplasm, the nucleus, etc.) that are accessible by the probes.

The cells may also not be processed according to histological techniques, but are adhered to other surfaces, including plastic, glass, polymeric surfaces such as gels, *etc*. Cells may be in a variety of states (such as formalin-fixed and/or paraffin-embedded), including frozen sections, live cells, cultured cells, cells subjected to suspension culture, etc.

Paraffin-embedded tissues on slides may be dewaxed using xylenes, as referenced in the experimental protocols provided herein. Any suitable organic solvent may be used, including xylene(s) benzene, toluene, and biphenyl. Further, the tissue slide may be placed in the organic solvent from 1 minute up to 30 minutes, or any range in between, or more. After dewaxing the sample can be rehydrated as necessary, using methods that are well-known in the art, including using solution containing decreasing concentrations of ethanol or another water-miscible solvent. Any suitable buffer may be utilized (e.g., Tris, etc.), in particular Citra Plus buffer.

### Antigen Retrieval

Formalin-fixed tissues sometimes require an antigen retrieval step before a probe can specifically bind to cells in the tissues. Cross-linking during fixation can mask antigenic sites and antigen retrieval methods can reverse, at least partially, this cross-linking and expose antigenic sites, allowing probes to bind. Methods of antigen retrieval are well known in the art. For example, the slides may be microwaved at maximal power (Sanyo, 1200 Watts) until boiling, wherein boiling may occur for any suitable amount of time, e.g., from 15 seconds up to 10 minutes or more, or any range in between. The slides may then be microwaved for an additional amount of time at medium power, wherein the amount of time may range from 1 minute up to 20 minutes, or more, or any range in between. The slides may be cooled for a period of time ranging from 5 minutes up to 30 minutes, or more, or any range in between, followed by rinsing with diH₂O.

A single heating step, or multiple heating steps may be used. Any combination or duration of heating steps, e.g., time, temperature, cycles, may be used provided that antigens are exposed and can be bound by a probe, such as a primary antibody, and that damage to the underlying biological material of the target cells is minimized

### Antigens

A variety of antigens may be used with the HMP method as described herein. In general, the HMP method may be applied to any biological target, e.g., including an extracellular target, a target on the surface of the cell, an intracellular target, a lipid, a protein, a cell type, a subcellular compartment, or any target that may be specifically labeled. A suitable antigen may be specific to a particular type of cell and/or may indicate a cancerous state of the cell (e.g., indicate high proliferation). For example, antigens associated with the presence of cancer include: alpha fetoprotein (AFP), CA15-3, CA27-29, CA19-9, CA-125, calcitonin, calretinin, carcinoembryonic antigen (CEA), CD34, CD99MIC 2, CD117, chromogranin, chromosomes 3, 7, 17, and 9p21, cytokeratin (e.g., TPA, TPS, Cyfra21-1), desmin, epithelial membrane antigen (EMA), factor VIII, CD31 FL1; glial fibrillary acidic protein (GFAP), gross cystic disease fluid protein (GCDFP-15), HMB-45, human chorionic gonadotropin, immunoglobulin, inhibin, keratin, lymphocyte marker, MART-1 (Melan-A), myo D1, muscle-specific actin (MSA), neuron-specific enolase (NSE), placental alkaline phosphatase (PLAP), prostate-specific antigen (PSA), PTPRC (CD45), S100 protein, smooth muscle actin (SMA), synaptophysin, thymidine kinase, thyroglobulin (Tg), thyroid transcription factor-1 (TTF-1), tumor M2, and vimentin.

A single antigen may be used with the HMP method provided herein, for example, an antigen unique to a specific type of immune cell. Alternatively, cocktails of antigens may be used, allowing isolation of multiple types of cells (e.g., the immune repertoire using CD 68, CD4, CD11, etc.). For example, a cocktail of probes may be used to recover multiple types of lymphocytes, as part of a diagnostic workflow to ascertain immunological activation status in a patient's cancer. As another example, the cocktail may comprise a mix of antibodies that bind to CD6, CD8, CD20, and CD68.

### Probes - Antibodies

In some embodiments of the method provided herein, the probe comprises an antibody that is specific for a particular antigen associated with a particular type of cell. The probe is conjugated or complexed with a reactive moiety comprising an enzyme capable of reacting with a substrate to produce a chemical material that is deposited on the cells to which the probe is bound. In general, the probe may comprise one or more antibodies, wherein at least one antibody is conjugated to an enzyme capable of reacting with a substrate to produce a protective chemical material. In some embodiments, under varying conditions, different amounts of chromogen (i.e., a chromogenic reagent) may be deposited onto the surface of the cell that are sufficient for a protective effect. If a plurality of probes is used, each type of probe may generate a light deposition of chromogen that in combination with depositions from other probes are sufficient for a protective effect.

In some embodiments, the probe comprises an antibody to a specific antigen, with the antibody complexed or conjugated to an enzyme capable of processing a substrate to form a protective chemical material. In other embodiments, the probe comprises a primary antibody that binds to a tissue antigen and a second antibody that binds to the primary antibody, where the secondary antibody is covalently conjugated or non-covalently complexed to an enzyme capable of processing a substrate to form a protective chemical material.

As an alternative (not part of the present invention), enzyme inhibitors (e.g., inhibitors to the enzyme used for degradation, such as proteinase K, trypsin, etc.) or other inhibitors of chemical processes may be complexed or conjugated to the probe, e.g., via the secondary antibody. Such enzyme inhibitor may locally deactivate or inhibit the degradation or processing of an enzyme or other reagent so that target cells, covered by the probe are protected. In cases in which the degradation enzyme is capable of penetrating the chromogenic stain, a polymer, e.g., polyethylene glycol (PEG), etc., may be conjugated or complexed with the degradation enzyme or other chemical reagent so that the enzyme or reagent is sterically hindered from penetrating the chromogenic stain, and thus, the underlying stained cells remain protected.

Essentially any antibody may be selected as a primary antibody, provided that it specifically binds to an antigen associated with a target cell type. Methods of determining antibody specificity are well known in the art. An antibody typically is considered specific if it binds to a target antigen without binding to unrelated antigens. In some embodiments, the primary antibody may bind to an antigen associated with a type of normal cell. In other embodiments, the primary antibody may bind to an antigen associated with a type of cancerous or tumorigenic cell. In still other embodiments, the primary antibody may bind to an antigen associated with a type of immune cell.

In some aspects, the antibodies may bind to antigens present on the surface of the cell, to extracellular antigens, or antigens present within the interior of the cell (provided that the cell was cross-sectioned during slide preparation).

Examples of suitable antibodies include: cytokeratin AE1/AE3 antibodies, anti-Ki-67 antibodies (Ki-67 is a nuclear antigen), anti-histone antibodies (histone is a nuclear antigen), anti-Epithelial Membrane Antigen (EMA) antibodies (EMA is a cell membrane antigen), anti-CD8 antibodies (CD8 is a lymphocyte antigen), anti-CD20 antibodies (CD20 is a lymphocyte antigen), anti-vimentin antibodies (vimentin is a stroma antigen), etc. In some aspects, cytokeratin AE1/AE3 antibodies may be a mixture of two different clones of anti-cytokeratin monoclonal antibodies, e.g., AE1 that detects high molecular weight keratins and AE3 that detects low molecular weight keratins. In other aspects, antibodies to mitochondrial antigens, antibodies to Golgi antigens, antibodies to nuclear antigens may be generated and used with the methods and systems provided herein.

Either a single probe or a cocktail of multiple probes can be utilized to protect cells, allowing flexibility and tunability with regard to experimental strategy. For example, in a histological section containing cancer cells, a probe can target the tumor cells during the micropurification process, thus separating the slide into two components; neoplastic cells versus all of the normal cell types (e.g., fibroblast, nerve, lymphocyte, etc.) and structural components (e.g., basal lamina, matrix, neo-vessels, etc.) present in the tumor microenvironment. If the probe(s) target tumor cells, then the normal cells and structural components are solubilized or otherwise processed, leaving behind the tumor cells. If a cocktail of probes target normal cells and structural components, then the tumor cells are solubilized or processed.

Suitable titers for antibodies include 1:10, 1:20, 1:50, 1:100, 1:200, 1:500, 1:1000, 1:2500, or any other amount.

In general, for antibody binding, any suitable incubation time may be used, provided that antigens exposed by antigen retrieval remain exposed and in a form in which antibody recognition and binding of the probe may occur. Examples of suitable incubation times for primary and/or secondary antibodies include 15 minutes, 30 minutes, 45 minutes, 60 minutes, or any range in between. In other aspects, incubation times with the secondary antibody may be 15 minutes, 30 minutes, 45 minutes, 60 minutes, or any range in between.

### Probes - Affimers

As another example, an affimer may be used as a probe. Affimers are small domain proteins that specifically bind to an antigen and typically exhibit high thermal and biophysical stability, and typically lack di-sulfide bonds or other post-translational modifications.

Affimers may be utilized with the HMP methods described herein. In some aspects, affimers may be biotinylated and detected using a streptavidin horseradish peroxidase (HRP) complex. For example, the horseradish peroxidase may oxidize DAB to produce a brown precipitate. In general, any suitable reporter molecule may be used.

### Probes - DNA

A further type of probe that may be used in the methods described herein is a chromogenic *in situ* hybridization (CISH) probe. CISH uses a complementary DNA or RNA strand to localize to a specific DNA or RNA sequence in a tissue specimen, and may be used to determine whether genetic mutations (e.g., gene deletion, chromosome translocation, gene amplification, etc.) are present. A combination of one or more antibody probes and one or more CISH probes allows identification and separation of cells and tissues that previously were difficult to visualize using the methods and systems described herein.

For example, a DNA probe may be generated that is complementary to a target DNA sequence of interest. The DNA probe may be labeled, for example with a digoxigenin label. A primary antibody can be used to bind to the probe (e.g., the primary antibody may be an anti-digoxigenin fluorescein isothiocyanate antibody). A secondary antibody may bind to the primary antibody, wherein the secondary antibody contains a reactive moiety capable of processing a reagent (e.g., the secondary antibody may be an anti-fluorescein-isothiocyanate horseradish peroxidase (HRP) antibody that reacts with DAB to produce and deposit a chromogenic signal (e.g., a dark brown precipitate) at the target site. Alternatively, the reactive moiety can be an alkaline phosphatase enzyme that is used to produce a chromogenic signal.

In general, the DNA probe may comprise any molecule that binds to a target molecule, wherein the probe is capable of reacting with a reagent to produce a chemical product that is deposited on the probe labeled cells.

### Chemical Material

In embodiments of the method described herein, a chemical material is deposited or placed on the surface of the target cells labeled with the probe to form a barrier. The material may be attached to the probe or may be a product of the probe (e.g., from processing a substrate). The chemical material may be soluble or insoluble. For example, the material may be generated as a product of a chemical reaction, wherein a reagent is processed by an enzyme attached to the probe to generate an insoluble product. Suitable enzymes include horseradish peroxidase and alkaline phosphatase.

Reagents are that react with such enzymes are generally referred to as chromogenic reagents. Suitable reagents for reaction with horseradish peroxidase include 3-amino-9-ethylcarbazole (AEC); 3,3'-Diaminobenzidine (DAB) (with or without a nickel enhancer); and Tetramethyl benzidine (TMB); Suitable reagents for reaction with alkaline phosphatase include 5-bromo-4-chloro-3indolyl-phosphate/nitrobluetetrazoleum (BCIP/NBT); Naphthol AS-MX phosphate + Fast Blue BB; Naphthol AS-MX phosphate + Fast Red TR; and Naphthol AS-MX phosphate + new fuchsin; and nitrobluetetrazoleum (NBT).

A secondary antibody used herein typically is conjugated to an enzyme which reacts with the chromogenic reagent to produce a chemical material. For example, glucose oxidase reacts with NBT to produce a blue insoluble chemical material; alkaline phosphatase reacts with naphthol AS-MX phosphate + new fuschin to produce a red insoluble chemical material, with naphthol AS-MX phosphate + fast red to produce a red insoluble chemical material, with naphthol AS-MX phosphate + fast blue BB to produce a blue insoluble chemical material, and with BCIP/NBT to produce a blue insoluble chemical material. Horseradish peroxidase reacts with TMB to produce a blue insoluble chemical material, with DAB (with or without a nickel enhancer) to produce a brown or black insoluble chemical material; and with AEC to produce a red insoluble chemical material. Lists of chromogens, and the enzymes that react with the chromogens may be found at, e.g., http://www.abcam.com/kits/chromogens-and-enhancers.

For example, the present techniques described herein may be used for ligand receptor binding studies. The precision of the HMP process may be used to localize the protective effect to selected ligand and/or bound receptors in a specific subcellular region, recovering only the biomolecules (e.g. signaling molecules) in the immediate vicinity that are associated with the receptor.

### Tunability

The method of the present invention is highly tunable, and may be configured to isolate a wide variety of targets, including types of normal cells, types of diseased cells (e.g., such as a cancerous or tumorigenic cell), as well as immune cells. Once isolated, the cells may be subjected to further analysis, including: NGS sequencing, expression profiling, proteomic analysis, lipid analysis, etc. In general, any target for which a suitable probe is provided, e.g., a probe which can specifically bind to the target and which is capable of reacting with a reagent to produce a protective barrier, e.g., a precipitate to cover the cell, may be isolated by the methods and systems provided herein.

In particular, the components employed in this process (e.g., probe, enzyme, chromogenic reagent, micropurification solution, buffers, etc.) may be individually tuned to a particular assay, and once the assay has been validated, the identified conditions (e.g., temperature, time, antigen retrieval processing, etc.) may be further optimized.

### Micropurification solution

The micropurification solution is used to degrade, digest or otherwise process cells that have not been protected by the protective barrier. The solution may contain at least one protease and, optionally, a detergent, and other enzymes and chemical reagents. For example, the micropurification solution, also referred to as a digestion buffer, may contain 1 mg/ml proteinase K and 2% SDS. When present, the SDS concentration may be from 0.01% SDS up to 5% SDS, or any amount in between.

The concentration of the protease used in the micropurification can be any concentration sufficient to degrade or process the cells not protected by the protective barrier. For proteinase K, the concentration may range from 0.1 mg/ml to 10 mg/mg, from 0.5 mg/ml to 5 mg/ml, or any amount in between. In some aspects the concentration of proteinase K is 1 mg/mg, 2 mg/ml, or 3 mg/ml.

For example, a slide is incubated in the digestion buffer for a time sufficient to digest the cells not protected with the barrier to the extent necessary to allow removal or processing of those cells. The incubation time may be 5 minutes, 10 minutes, 15 minutes, 30 minutes, 45 minutes, 60 minutes, 75 minutes, 90 minutes, 120 minutes or 150 minutes or any amount in between. For longer incubation times, additional buffer or diH₂O may be added so that the slides do not dry out. In some aspects, digestion/processing during micropurification may be complete in 10-20 minutes.

In general, any serine protease may be used, including proteinase K, trypsin, chymotrypsin, etc. In some embodiments, the micropurification solution comprises one serine protease, two serine proteases, or three or more serine proteases. In still other embodiments, degradation enzymes may be selected based upon the type of tissue to be analyzed, e.g., enzymes known to degrade muscle tissue and cells may be selected for slides containing muscle tissue, enzymes known to degrade adipose tissue and cells may be selected for slides containing adipose tissue, etc.

In general, any suitable detergent may be used, including SDS, ADS, Triton X-100, Sarkosyl, and Tween. Further, in some aspects, two or more detergents may be used as part of the micropurification solution.

In general, any suitable buffer may be used, including Citra Plus, Tris-HCl, and citrate buffer. Further, in some aspects, two or more buffers may be used as part of the micropurification solution.

The pH of the micropurification solution may range anywhere from a pH of 1 to a pH of 10. In general, the pH should match the optimal conditions of the micropurification enzyme.

The barrier or shield may prevent, inhibit, or reduce degrading components present in the micropurification buffer (e.g., proteases, detergents, etc.) from physically contacting the targeted cells. In other embodiments, the barrier may act to inhibit or reduce activity of a degrading enzyme, e.g., such as a protease. Alternatively, the barrier may restrict or confine motion of the protected biological material to prevent or reduce exposure to the degrading enzymes.

The method provided herein may be used to identify novel general or patient specific biomarkers. For example, analysis of the digested, processed, or solubilized portion of the slide may lead to the identification of novel serum biomarkers or validation of predicted biomarkers. Solubilized protein from microprotected cells can be analyzed via SDS-PAGE, western blot, reverse phase protein arrays, dot blots, mass spectrometry, etc.

### Kits (no part of the present invention)

Kits for carrying out the method of the present invention may comprise any or all of the reagents necessary to carry out the methods described herein. The kit may comprise a micropurification solution, and optionally, a neutralization solution that deactivates enzymes present in the micropurification solution to halt micropurification. The kit may also comprise individual reagents which are combined to form the micropurification solution.

The kits may comprise a probe and a chromogenic reagent, which reacts with the probe to generate an insoluble chemical material, along with the micropurification solution. The probe may be a monoclonal antibody or other antigen binding molecule, which specifically binds to an antigen present in the biological sample. The probe may be conjugated to an enzyme capable of reacting with the reagent to produce the insoluble chemical material.

Kits may also comprise the micropurification solution, a second antibody (or a second antigen binding fragment thereof) that specifically binds to a first monoclonal antibody (or a first antigen binding fragment thereof). In this example, the first monoclonal antibody may be custom generated, and designed to bind to the second antibody. Custom mAbs may be produced, e.g., to bind to a specific marker associated with a cell, depending on the nature of the assay. The second antibody is conjugated to or complexed with an enzyme, which reacts with the substrate (i.e., the chromogenic reagent) to produce a protective barrier. For example, the second monoclonal antibody may be biotinylated to facilitate binding of enzyme-labeled streptavidin, where the enzyme produces an insoluble chemical material. Kits may also include the chromogenic reagent, e.g., a chromophoric substance including DAB and Fast Red, which is processed by the second antibody. The probe may additionally comprise a first monoclonal antibody, specific to a molecule present in the biological sample.

If it is desirable to inactivate proteinase K, the kit may include a neutralization solution comprising a protease inhibitor such as PMSF or AEBSF (Pefabloc^{®}) to permanently inactivate proteinase K.

In other embodiments, a kit may contain one or more of the following reagents for immunostaining and microprotection, including:
Reagent 1: non-aqueous mounting medium (e.g., Xylene)
Reagent 2: serum blocking reagent
Reagent 3: primary antibody diluent reagent (may comprise BSA)
Reagent 4: biotinylated secondary antibody
Reagent 5: streptavidin-enzyme conjugate
Reagent 6: Chromogen
Reagent 7: chromogen buffer (*e.g*., may contain other reagents such as 0.1% hydrogen peroxide)
Reagent 8: chromogen neutralization solution (e.g., up to 30% hydrogen peroxide) Reagent 9: Counter stain (*e.g*., hematoxylin)
Reagent 10: Microprotection Solution
Reagent 11: Microprotection Neutralization Solution

Reagents may be provided in concentrated form. A primary mAb may be provided with one or more of Reagents 1-11. Alternatively, a primary mAb may be provided with one or more of Reagents 2-8.

The HMP kit for biomolecule analysis (*e.g*., including analysis for DNA, RNA, lipid, protein, metabolite, *etc*.) from cells obtained from clinical or animal model tissue specimens may at least comprise a micropurification solution. Depending upon the usage for the particular type of kit, additional reagents may be provided to facilitate analysis of a particular biological sample, e.g., mRNA, miRNA, and proteomic kits, which may or may not be optimized according to the techniques provided herein. Solutions and protocols optimized for each biomolecule and application are provided herein.

The HMP kit can be used with commercial IHC kits. For instance, IHC may be carried out using a commercial kit (*e.g.,* http://zyagen.com/userfiles/ Zyagen%20Peroxidase%20IHC%20Kits.pdf), and once staining is complete, the micropurification solution may be added to capture target and non-target cells.

### TaqMan^{™} assays

Once isolated by using the method of the present invention, the target cells may be subject to further analysis, e.g., DNA analysis or gene expression assays, such as a TaqMan^{®} assay. A variety of TaqMan^{®} gene expression assays are available (e.g., ThermoFisher's TaqMan^{®} Gene Expression Assay, http://www.thermofisher.com/us/en/home/life-science/pcr/real-time-pcr/real-time-pcr-assays/taqman-gene-expression.html). Nucleotide hybridization probes may be obtained that specifically hybridize to DNA comprising the mutation of interest. In other aspects, custom hybridization probes may be designed in order to screen for particular mutations.

### Advantages

In addition to being widely accessible due to its simplicity and low cost, the method of the present invention described herein provide a significant improvement in precision compared to current methods. While laser dissection instruments have been used to perform ultra-precise procurement in the past, e.g., recovering individual nuclei, this is still an extremely challenging undertaking that typically retrieves only a few targets due to the tediousness of the human-based targeting process. For most downstream molecular assays, procuring a large number of cells or organelles is advantageous [23, 35, 36, 47-49], since the reproducibility and robustness of data sets generally improve as the amount of starting material increases.

Present techniques allow for moderate- and low-abundant molecules (miRNAs, mRNAs) and DNA allele variants to be isolated and even measured. This is important for assays such as proteomic assays wherein the dynamic range of expression is many orders of magnitude. Because micropurification is based on a molecular probe for targeting, all stained cells, organelles, or nuclei are rapidly retrieved when the histological slide is processed, whether that is one cell/organelle/nuclei or one million cells/organelles/nuclei. Thus, relatively large amounts of DNA, RNA or protein from individual cells or from subcellular structures can be recovered for molecular analysis using the micropurification process.

Micropurification may be used to select and purify target and non-target cells, organelles and nuclei, as well as other structures, facilitating further analysis of a variety of different cell types and biological molecules including DNA, RNA, proteins, lipids, metabolites, etc. The protocols and components/reagents may be tuned and optimized to isolate a wide variety of different types of cells (e.g., normal and diseased), and biological molecules. Such techniques also may be used to quantitatively validate the robustness and reproducibility of mutation testing, and to extend measurement capability to labile components such as mRNA and miRNA.

The method of the present invention provides for a rapid, easy-to-use, precise (at cellular level) and inexpensive cell procurement system that facilitates tumor mutation analysis for patient care and personalized medicine. The method is also suitable for laboratory-based research, and does not rely upon sophisticated instrumentation, such as that found with laser dissection instruments. Accordingly, the method may readily be performed in a variety of settings, including research labs, field applications, remote medical locations etc.

The methods provided herein may be used to test for the presence of a variety of different types of tumor mutations. A series of histology samples may each be screened for the presence of a particular tumor marker. Also, cancer cells may be isolated, and the corresponding genetic material subjected to NGS to determine a mutation profile for a particular patient having a particular type of cancer.

Processing may occur in parallel to facilitate high throughput analysis. Thus, a plurality of slides may be individually screened for the presence of a mutation or a protein in a manner of minutes (e.g., in 10-20 minutes or more). The isolated cells having the protein or mutation may undergo further processing.

The method provided herein may be performed manually. The method may also be incorporated into an automated system for processing histology slides, as human intervention or complex instrumentation is not needed to isolate target cells.

The HMP method provided herein may be combined with microdissection, genomic analysis, and proteomic analysis. Such techniques can be used to facilitate personalized medicine, e.g., to quickly isolate a type of patient cell having a particular mutation or expressing a particular protein, wherein the isolated cells can undergo additional types of analysis, e.g., NGS, expression analysis, expression profiling, proteomic analysis, etc., to generate a personalized profile for a particular patient.

Applications tailored to RNA studies may be developed using the techniques provided herein. While antigen retrieval may make non-target cells more susceptible to complete digestion and removal from the slide, this process may also hydrolyze RNA of both the target and non-target cells. As an alternative method, the micropurification solution may comprise RNase as the degrading enzyme to digest RNA in the non-target cells. For example, the protective barrier will prevent RNase from degrading RNA of protected cells. If the RNase is able to penetrate the protective barrier, the RNase may be modified, e.g., to be tethered to a polymer to physically block passage through the barrier. The protected cells may then be collected, and their respective RNA obtained for further analysis. In this example, DNA, proteins, or other materials present in the non-target cells do not need to be removed (i.e., complete digestion is not needed) since the downstream molecular assay for RNA analysis is specific to RNA.

Specific examples of HMP protocols are provided below tp further illustrate the present invention.

### EXAMPLES

### Example 1: Methods of Micropurification of Target Cells

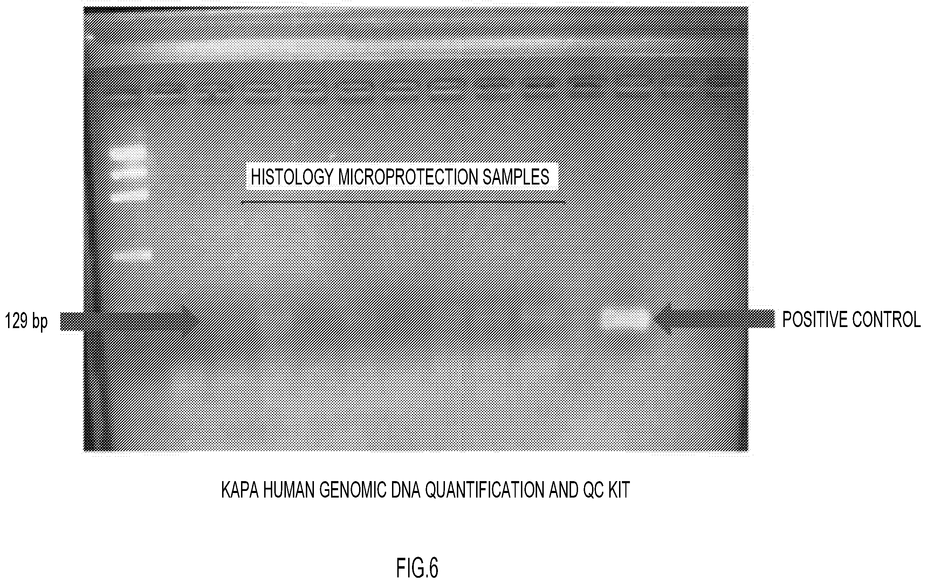

Example protocols for analyzing target cells are provided herein. This example also relates to FIGs. 5A-5F and FIG. 6. An immunohistochemical staining procedure is provided, which protects a target cell population from a micropurification solution, but does not protect unstained non-target cells. Enzymatic treatment of the stained tissue section digests the non-target cells into the micropurification solution (digestion buffer). The slide may be washed, and the target cells retrieved from the slide for molecular analysis.

### Preparation

In some cases, H&E stains were initially performed on tissue sections to assess morphology and cellular content. The microtissue slide was placed in xylenes for about 10 minutes, and was rehydrated using graded alcohols and water. Slides were placed in a staining rack within a Tissue Tek staining dish, filled with 250 ml of 1x Citra Plus buffer (BioGenex), and covered loosely.

### Antigen Retrieval

After preparation, antigen retrieval was performed. Antigen retrieval involves exposing the surface of the cells, e.g., by melting polymer that may cover the cells during paraffin embedding and fixation of the biological sample onto the histology slide. Once exposed, antibodies or other molecular probes may contact the biological sample, e.g., to bind to markers, proteins, receptors, nucleotide sequences, etc. For slides embedded in paraffin, antigen retrieval may help induce or enhance susceptibility of the sample to micropurification.

The slides, covered by Citra Plus buffer, were microwaved for 5 minutes at high power (Sanyo, 1200 Watts) until boiling. The buffer level was checked and diH₂O was added, as needed, to keep the tissue sections moist throughout the procedure. The slides were then microwaved for 15 minutes at power level 3, the buffer level was inspected halfway through the 15 minutes, and diH₂O was added as needed. The slides were allowed to cool in the microwave for an additional 20 minutes. Finally, the slides were rinsed with diH₂O.

### Labeling

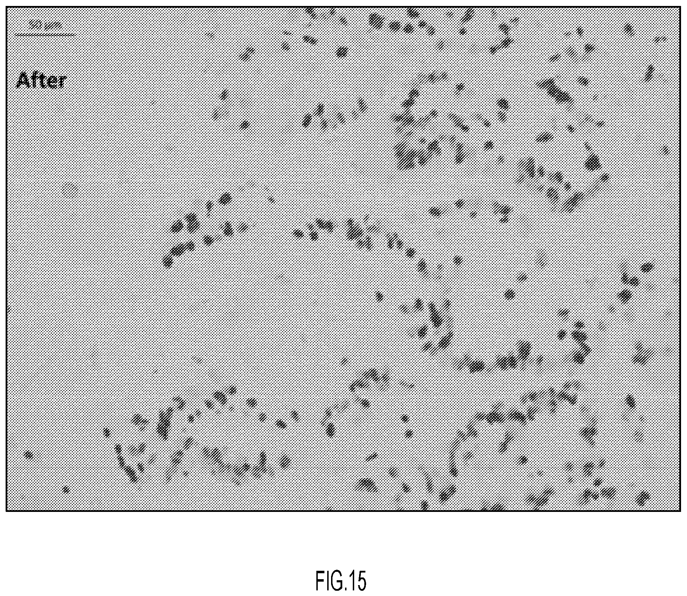

Following antigen retrieval, the slides were incubated at room temperature for about two hours with primary antibody, for about one hour with secondary antibody, and then detection was completed using DAB chromogen with no counterstain. The primary antibodies used for immunohistochemistry were cytokeratin AE1/AE3 mAbs (1:100, BioGenex) and anti-Ki-67 mAbs (1:100, BioGenex) (see, *e.g.,* FIGs. 5A-5F, FIG. 14A-14B, and 15). After DAB deposition, slides were rinsed in water and imaged using an AxioLab.A1 microscope and Axiocam 105 camera.

### Microprotection

Slides were incubated in 200-300 microliters of micropurification solution (also referred to as digestion buffer) containing 1 mg/ml proteinase K and 0.1% SDS for 30 mins at 56°C. The digestion step dissolved away the non-microprotected cells and extracellular structures (i.e., the non-stained cells and structures). The micropurification solution was decanted, and the slides were washed three times in PBS and imaged, followed by scraping of the microprotected cells that remained on the slide into a fresh PBS buffer solution for molecular analysis. The target cells, recovered from the slide by manual scraping, were analyzed, demonstrating that the DNA in the target cells is present and amenable to PCR amplification (*see, e.g*., FIG. 6).

In some cases, immunohistochemical staining was performed before and after HMP. FIGs. 5A-5F show a series of microscopic images for histological sections of multiple samples of colorectal cancer before (see, e.g., FIG. 5A and 5D) and after (see, e.g., 5B, 5C, 5E and 5F) HMP. Immunohistochemical staining of a histology section from several patients having colorectal cancer produced a dark DAB precipitate over the tumor cells (labeled with an anti-cytokeratin mAb) or over the tumor cell nuclei (labeled with an anti-Ki 67 mAb) as shown in FIG. 5A-5F. Subsequent incubation of the histological section with a buffer containing proteinase K and SDS digested the non-target cells off the slide but left the targeted tumor cells intact, in effect, separating the tumor cells from the non-tumor cells.

### Molecular Analysis of DNA

DNA was extracted from the recovered cells via overnight incubation with proteinase K at 65°C with shaking (500 rpm). Samples were then heated at 95°C for 15 minutes to inactivate proteinase K. PCR with the extracted DNA samples was performed with the KAPA Human Genomic DNA Quantification and QC Kit for the 129 bp fragment. Forty PCR cycles were completed with each cycle at 95°C for 10 seconds and 62°C for 30 seconds. Amplified DNA samples were run on a 2% TBE agarose gel with ethidium bromide staining. The gel was imaged on a UVP ChemiDoc-IT.

Similar techniques may be employed to recover and analyze additional biomolecules in the target cell(s)/structure, including RNA, proteins, lipids, metabolites, and others. For example, the isolated cells may be subjected to RNA analysis, e.g., using a RNeasy kit from Qiagen to obtain RNA. The isolated cells may be subjected to protein analysis, e.g., using a ReadyPrep protein extraction kit to capture proteins from Bio-Rad. In still other aspects, Abcam's lipid extraction kit may be used to isolate lipids. Accordingly, once isolated, the cells may be subjected to a variety of different types of analysis to recover and analyze biomolecules in the target cell(s)/structure, including RNA, proteins, lipids, metabolites, and so forth.

### Example 2: Methods of Purification of Non-target cells

Example protocols for analyzing non-target cells are provided herein. An immunohistochemical staining procedure is provided, which protects a target cell population from a micropurification solution, but does not protect unstained non-target cells. Enzymatic treatment of the stained tissue section digests the non-target cells into the micropurification solution (digestion buffer) and allows the non-target cells to be collected for molecular analysis.

### Preparation

In some cases, H&E stains were performed on tissue samples to first assess morphology and cellular content. Immunohistochemistry staining was completed after placing the slide in xylenes for 10 minutes and rehydrating the slides using graded alcohols and water, prior to antigen retrieval.

Slides were placed in a staining rack within a Tissue Tek staining dish, filled with 250m1 of 1x Citra Plus buffer (BioGenex), and covered loosely.

### Antigen Retrieval

The slides, covered by Citra Plus buffer, were microwaved for 5 minutes at high power (Sanyo, 1200 Watts) until boiling. The buffer level was checked and diH₂O was added, as needed. The tissue sections were kept moist throughout the procedure. The slides were then microwaved for 15 minutes at power level 3 and the buffer level was inspected halfway through the 15 minutes and diH₂O added as needed. The slides were allowed to cool in the microwave for an additional 20 minutes. Finally, the slides were rinsed with diH₂O.

### Labeling

Following antigen retrieval, the slides were incubated at room temperature for two hours with primary antibody, for one hour with secondary antibody, and then detection was completed using the iView kit and DAB chromogen with no counterstain. The primary antibodies used for immunohistochemistry were cytokeratin AE1/AE3 mAbs (1:100, BioGenex) and anti-Ki67 mAbs (1:100, BioGenex). After DAB deposition, slides were rinsed in water and imaged using an AxioLab.A1 microscope and Axiocam 105 camera.

### Microprotection

Slides were incubated in 200-300 microliters of micropurification solution (digestion buffer) containing 1 mg/ml proteinase K and 0.1 % SDS for 30 minutes at 56°C. The digestion step solubilized the non-microprotected cells and structures (i.e., the non-stained cells), which were collected in the digestion buffer for subsequent molecular analysis.

### Molecular Analysis

Immunohistochemical staining of a histology section from several cases containing colorectal cancer produced a dark DAB precipitate over the tumor cells (cytokeratin) or over the tumor cell nuclei (Ki-67) as shown in FIGs. 5A-5F. Subsequent incubation of the histological section with the buffer containing proteinase K and SDS digested the non-target cells off the slide but left the targeted tumor cells intact, in effect separating the tumor cells away from the non-tumor cells. The digested cells/structures were recovered from the slide in the digestion buffer for subsequent analysis.

### Example 3: Optimization of Cell and Nuclear Enrichment

Mixtures of cultured cells with known genomic status are prepared to: (a) quantitatively assess the efficiency of target cell enrichment of cells and nuclei; and (b) optimize the HMP process by varying one or more of the purification components, temperature, time, slide preparation, and IHC conditions in order to determine optimal conditions.

### Cell Block and Slide Preparation

Human tumor cell lines, such as Burkitt lymphoma ST486 (KRAS and BRAF wild-type), lung carcinoma A549 (KRAS+ homozygous mutation), and melanoma UACC.62 (BRAF+ heterozygous mutation) were obtained from the ATCC. Cells were grown to near confluence in T-125 flasks and recovered into PBS buffer. ST486 cells were admixed with A549 or UACC.62 at 1:100, 1:10, 50:50, 10:1, and 100:1 ratios. Each admixed sample was processed into a formalin-fixed, paraffin-embedded cellblock. Histological sections from each block were sliced at 4-, 10-, and 25- micron thickness. Sections were unbaked or baked at 60°C for 20 minutes, dewaxed, and rehydrated through xylenes and graded alcohols, using a short (2 min per step) or long (10 min per step) protocol. Triplicate recut sections are prepared from each block.

### Antigen Retrieval

Slides were placed in a staining rack in a Tissue Tek staining dish filled with 250 ml of 1x Citra Plus buffer (BioGenex). The slides were microwaved for 5 minutes at maximal power (Sanyo, 1200 Watts) until boiling, and microwaved for an additional 15 minutes at medium power, then cooled for 20 minutes and rinsed with diH₂O.

In parallel, additional slides were processed through a range of heating times, temperatures, and buffer conditions.

### Labeling

Slides were incubated at room temperature for 30 minutes with a primary antibody (using a range of titers), for 30 minutes with a secondary antibody, and then detected using DAB or Fast Red or another suitable chromogen. Select slides were counterstained using IHC (e.g., with H&E) and rinsed in water.

Slides may be incubated at or about room temperature for any amount of time ranging from 1 to 60 minutes for the primary antibody, and from 10 to 60 minutes with the secondary antibody.

For example, cell targeting antibodies may bind to cytokeratin, melanoma antigen recognized by T cells (MART-1), or transcription termination factor 1 (TTF1) while nuclear targeting antibodies bind to Ki-67 or histone, each of which has been successfully performed in previous studies [50].

### Micropurification

Slides were incubated in 200-300 microliters of micropurification solution for 10 mins at 56°C. The incubation time may vary anywhere from 1 minute to 30 minutes, or even 60 minutes. Conditions to be assessed include varying the major components of the purification solution (e.g., detergents, proteases, additional enzymes, buffers, and pH, etc.) by varying the amounts of one component, two components, three components, four components, five components, and so forth during each processing run, as well as by varying the time and temperature of the processing run.

Non-stained cells and structures were recovered for downstream molecular analysis. The slides were washed and imaged. After imaging, the stained target cells/structures were retrieved for subsequent analysis.

### Imaging

Digital imaging of the histological sections may be performed to quantitate procurement efficiency using an AxioLab.A1 microscope and Axiocam 105 camera. The purity of the isolated cells and nuclei was assessed by both manual and auto-counting. Triplicate serial recut sections were evaluated for reproducibility.

### DNA Analysis

DNA was extracted from the recovered cells and nuclei after overnight incubation with proteinase K at 65°C with shaking (500 rpm). Samples were heated at 95°C for 15 minutes to inactivate proteinase K.

Quantitative PCR (qPCR) with the extracted DNA was performed using the KAPA Human Genomic DNA Quantification and QC Kit for the 305 bp, 129 bp and 41 bp fragments. Forty PCR cycles were run with each cycle at 95°C for 10 seconds and 62°C for 30 seconds. All assays were completed in triplicate. The Q129/Q41 and Q305/Q41 ratios were calculated to determine DNA quality.

For cell enrichment, Taqman qPCR may be used to measure the mutant and wild type allele ratio in each cell admixture sample (*KRAS* and *BRAF* wild type; *KRAS+* mutation, *BRAF+* mutation). For nuclear enrichment, Taqman qPCR was used to measure genomic DNA and mitochondrial DNA in whole cells versus purified nuclei, and the ratio is assessed for each set of micropurification conditions [51-54].

To measure quality of the assay, DNA KAPA Q-ratios may be generated which indicate the quality of the recovered DNA. Target cells and nuclei were procured with >95% purity and >95% efficiency based on analysis of the digital images of the histology slides. The purity of target cells and nuclei was >95% based on analysis of the DNA mutation data and the ratios of genomic to mitochondrial DNA, respectively. Slide-to-slide reproducibility for cellular and nuclear enrichment was >95%. Thus, the quality was sufficient for standard DNA allelotyping and sequencing assays that are routinely performed in the clinic and research laboratory, and can be carried forward to future R&D and commercialization efforts.

Protocols similar to the micropurification protocol used for *KRAS* gene mutation analyses may be used to analyze additional types of tumor mutations in cells.

By optimizing the purification process, as described herein, the cells may be purified to a level sufficient to meet the proficiency standards of a clinical assay in a CLIA laboratory.

### Example 4: mRNA and miRNA Analyses

The status of mRNA and miRNA after micropurification and optimization was evaluated to determine whether quantitative reverse transcriptase PCR (qRT-PCR) analysis can be performed.

### Cell Block and Slide Preparation

Anonymized human tumor specimens were obtained from the Department of Pathology at Sinai Hospital, Baltimore, MD. Histological sections from each block were sliced at 4-, 10-, and 25-micron thickness. Sections were unbaked or baked at 60°C for 20 minutes, dewaxed, and rehydrated through xylenes and graded alcohols, using a short (2 min per step), intermediate (5 min per step), or long (10-15 min per step) protocol. Triplicate recut histological sections were prepared from each block.

### Antigen Retrieval

Slides were placed in a staining rack within a Tissue Tek staining dish, filled with 250ml of 1x Citra Plus buffer (BioGenex), and covered loosely. One set of slides was microwaved using standard conditions comprising: microwaving the slides for 5 minutes at maximal power (Sanyo, 1200 Watts) until boiling, microwaving the slides for an additional 15 minutes at medium power, and then cooling the slides for about 20 minutes and rinsing with diH₂O.

In parallel, additional slides were processed through a range of heating times, temperatures, and buffer conditions. To identify optimized conditions, parameters (e.g., processing times, concentrations, temperatures, etc.) may be varied 10X higher and 10X lower from condition(s) that produce the desired effect, e.g., microprotection of target cells under conditions that do not lead to substantial degradation of the underlying biological material to be analyzed (e.g., by DNA analysis, RNA analysis, protein analysis, lipid analysis, etc.).

### Labeling

Slides were incubated at room temperature for 30 minutes with primary antibody, for 30 minutes with secondary antibody, and then stained with DAB or Fast Red chromogen with no counterstain. After DAB deposition, the slides were rinsed in water and imaged using an AxioLab.A1 microscope and Axiocam 105 camera to document IHC staining quality.

Slides may be incubated at or about room temperature for any amount of time ranging from 1 to 60 minutes for the primary antibody, and from 10 to 60 minutes with the secondary antibody.

### Micropurification

Slides were incubated in 200-300 microliters of a purification solution for 10 mins at 56°C. The incubation time may vary anywhere from 1 minute to 30 minutes, or even 60 minutes. 1 mg/ml proteinase K in Tris buffer with 1% SDS may be used as the micropurification buffer. Conditions may be varied, and therefore, conditions to be assessed include all of the major components of the purification solution (e.g., detergents, proteases, additional enzymes, buffers, pH, etc.) by varying the amounts of one component, two components, three components, four components, five components, and so forth during each processing run, as well as by varying the time and temperature of the processing run.

Non-stained cells and structures were recovered for downstream molecular analysis. The slides were washed and imaged. After imaging, the stained target cells/structures were retrieved for subsequent analysis.

The process was repeated on three recut histological sections to assess reproducibility, and the slides are imaged using an AxioLab.A1 microscope and Axiocam 105 camera to document the status of micropurification.

### mRNA and miRNA Analysis

mRNA and/or miRNA were extracted from the micropurified cells samples using Qiagen RNeasy^{®} and Qiagen miRNeasy FFPE^{®} kits, respectively, according to the manufacturer's instructions.

Initial quality and quantity assessment of the extracted RNA was determined with a Qubit RNA IQ assay using a Qubit 4 Fluorimeter (ThermoFisher Scientific). For quantity assessment of miRNA samples, the Qubit microRNA Assay Kit was used. The extracted RNA was converted to cDNA using a High Capacity cDNA Reverse Transcription Kit (ThermoFisher Scientific). 10 ng of RNA were used for each reaction. The thermal cycler was set for the following: 25°C for 10 minutes, 37°C for 120 minutes, 85°C for 5 minutes.

qRT-PCR was performed on a StepOnePlus instrument (ThermoFisher Scientific). Two human endogenous controls [Human ACTB (Beta Actin) and Human GAPD (GAPDH)] were used to assess RNA levels across samples. Forty PCR cycles were run with each cycle at 95°C for 1 second and 60°C for 20 seconds. All assays were completed in triplicate. For miRNA analysis, the TaqMan Advanced miRNA Human Endogenous Control 96-well plate is used. cDNA template was generated according to the manufacturer's instructions prior to running the assay.

All micropurified samples are compared to macrodissected (i.e. scraped) immunostained serial recut tissue sections to evaluate the impact of the micropurification process on RNA/miRNA quality and quantity.

In some embodiments, biomolecule hydrolysis may occur during antigen retrieval or IHC staining, for components such as mRNA/miRNA that may be more labile. The protocol (e.g., antigen retrieval and/or IHC staining) may be adjusted to reduce or eliminate hydrolysis.

The tunability of the method of the present invention is a particular strength of micropurification. Multiple options are available (e.g., one could use different reagents for the labels or for the micropurification solution, or one could use different experimental conditions for the micropurification process (e.g., different temperatures, duration of processing steps, etc.) for completing the micropurification process. Accordingly, the protocol may be adjusted to avoid nucleic acid hydrolysis, if needed.

### Detection of Beta-Actin and GAPDH transcripts

Detection of beta-actin and GAPDH transcripts in the 20-30 Ct level was achieved using qRT-PCR after the micropurification process. Detection of miRNA endogenous control panel genes in the 20-30 Ct level was achieved after the micropurification process. A range of values was expected depending on the abundance of each miRNA in the target cells and subcellular structures, and thus, the level of each miRNA was carefully compared between the purified samples and the tissue scrape controls to ensure all expressed miRNAs are successfully measured using the new technology.

### Example 5: Micropurification Kit for Cells

Micropurification kits (no part of the present invention) may be provided to isolate specific types of cells without using complex instrumentation. For example, a micropurification kit may allow rapid and inexpensive procurement of specific types of cells using the techniques provided herein. Once the cells have been isolated, and thus, purified from the biological sample (e.g., from clinical or animal model tissue specimens affixed to a histological slide), the isolated cells may be subjected to further analysis, e.g., DNA analysis, RNA analysis (including mRNA, miRNA, etc.), proteomic analysis, etc. Such analyses may be used to build a profile of the specific type of isolated cell. For example, such kits may facilitate tumor mutation analysis for patient care and personalized medicine. For each type of kit, solutions and protocols may be optimized for each biomolecule type and application.

Such kits may utilize an immunohistochemical staining procedure to label a target cell population with an insoluble chemical material that protects the labeled and stained cells. Subsequent treatment with a micropurification solution, which includes one or more enzymes which digest the non-stained cells of the tissue section, allows for collection and molecular analysis of target cell contents.

### Example 6: Micropurification of Clinical Samples using DAB

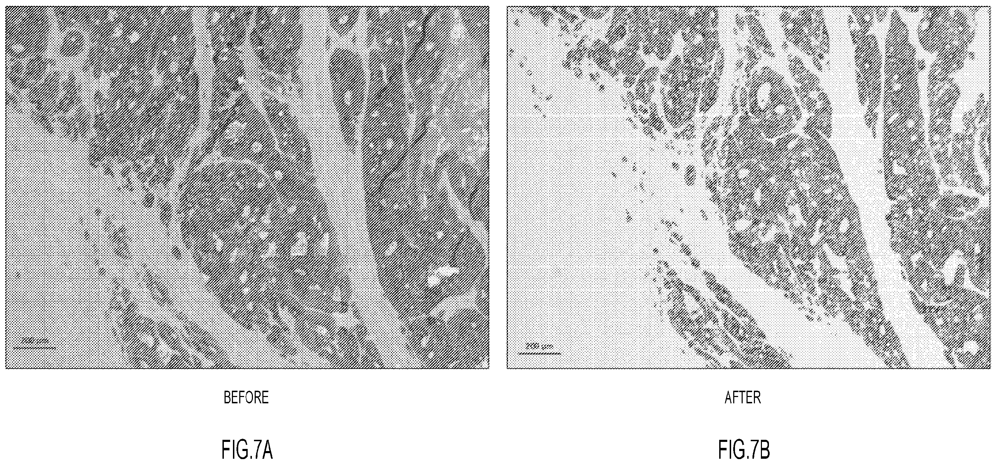

The micropurification process provided herein was performed on clinical samples. FIGs. 7A - 9B show invasive colorectal cancer and lymphocytes before and after immunohistochemical (IHC) staining.

FIGs. 7A and 7B show two serial histological sections of colon carcinoma both stained with cytokeratin AE1/AE3 mAb antibodies followed by deposition of 3,3'-diamino benzidine (DAB) chromogen (at a 50X magnification) onto the labeled cells. The section of FIG. 7A was also counterstained with hematoxylin and eosin (H&E) to allow visualization of the nontarget cells/structures (stroma, fibroblasts, inflammatory cells, nerves), which appeared as pink or red, and are shown as lightly stained in the grayscale image. Thus, FIG. 7A shows the histological section before micropurification solution.

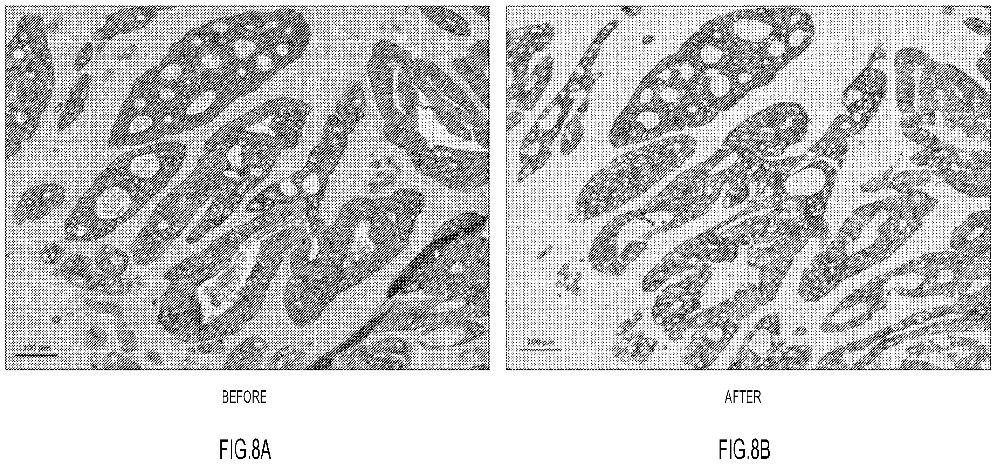

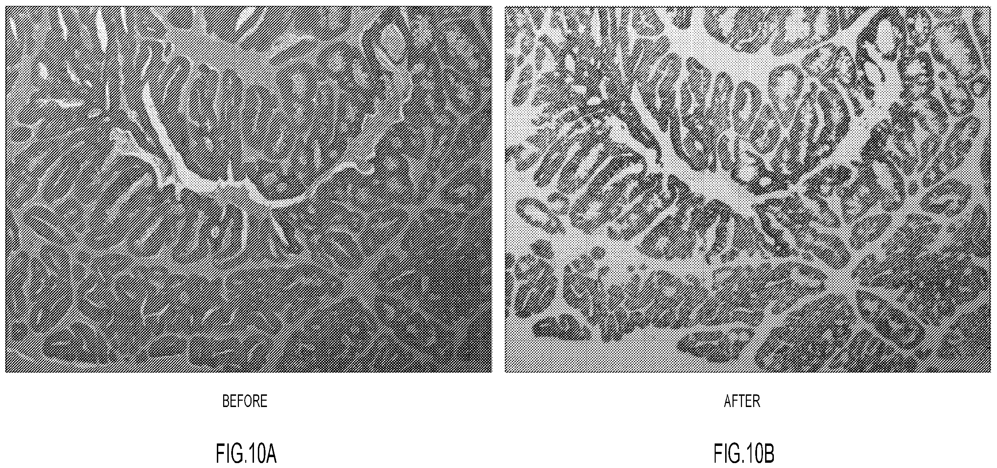

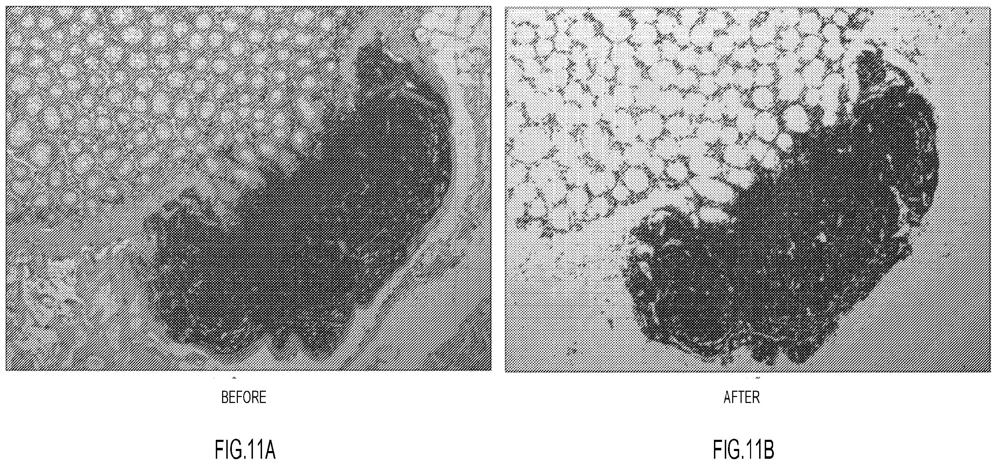

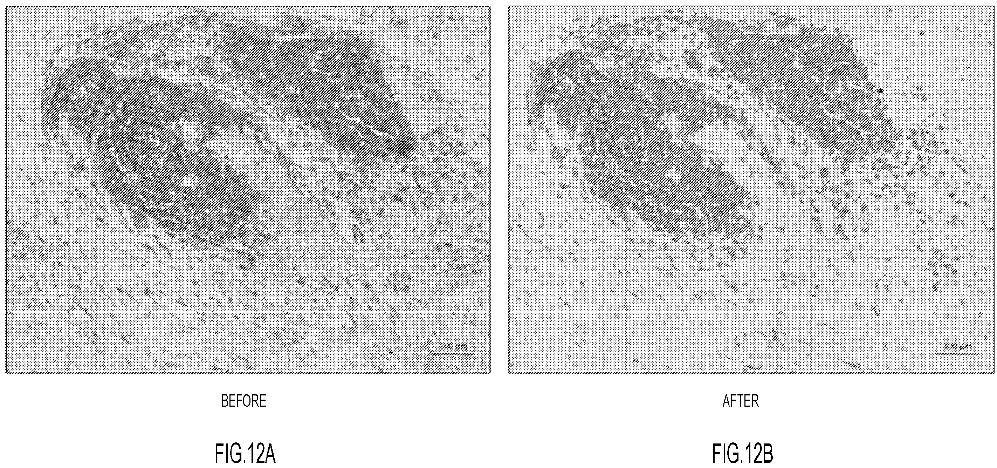

The section of FIG. 7B was subjected to micropurification and similarly counterstained with H&E. The target cells remain intact on the slide but the non-targets are solubilized and no longer are visible. Thus, FIG. 7B shows the histological section after the micropurification solution was added, as the non-target cells are no longer present. In both FIGs. 7A and 7B, the stained cancer cells were darkly colored (shown as darkly stained in the grayscale image). Similar experiments were performed on a variety of histological sections, as shown in FIGs. 8A-14B.

High-power microscopic visualization was used to verify that the process strips the non-stained areas down to glass with no remaining cells, organelles, or other structures. For example, FIGs. 8A and 8B show a similar colon carcinoma stained with the insoluble chemical material before and after purification at 100X magnification.

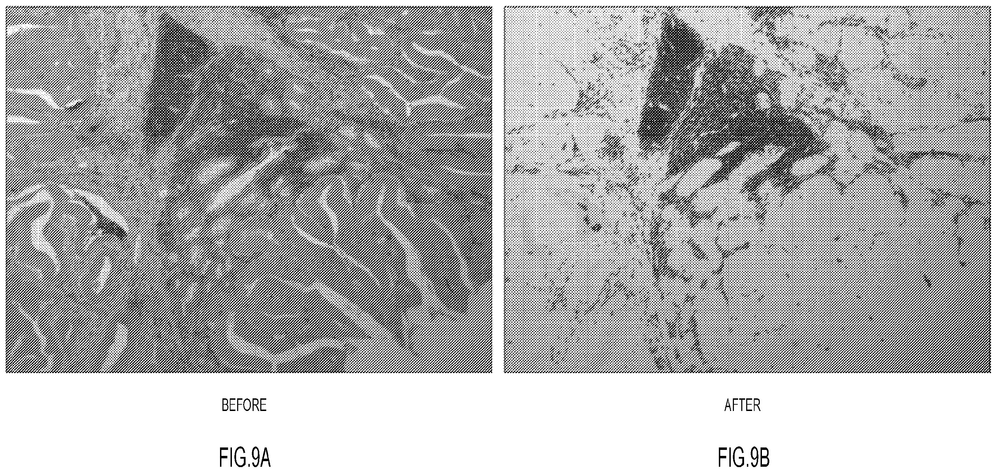

FIGs. 9A and 9B show images of a colon carcinoma with areas of significant chronic inflammation as shown by staining with a panlymphocyte antibody cocktail against CD8, CD20, and CD60 (50X magnification), which are inflammation markers. The lymphocyte population consisted of foci of intense inflammation in the upper middle part of the slide, with thin bands of lymphocytes streaming through the stroma, and scattered individual lymphocytes elsewhere throughout the section (FIG. 9A). After the micropurification process, a pure population of lymphocytes remained on the slide, including the inflammatory foci, lymphocyte bands, and individual cells (FIG. 9B).

### Example 7: Micropurification of Clinical Samples Using Fast Red

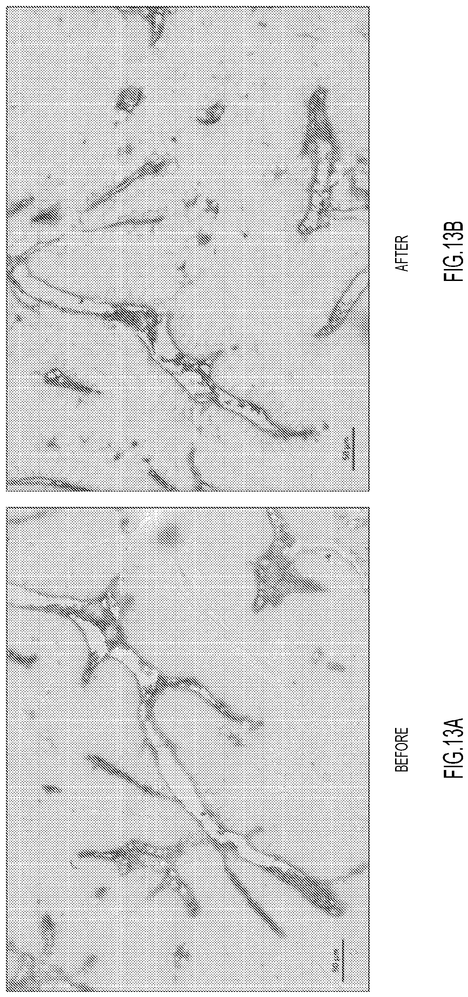

As another example, involving purification of membranes, colon cancer was stained with an antibody against epithelial membrane antigen (EMA), using Fast Red as the chromogen instead of DAB (FIG. 13A-14B). The tumor cells in FIG. 13A had a light pink blush covering the cytoplasm, with strong membrane staining associated with luminal structures. After micropurification, the non-stained tumor cell and extracellular matrix components were completely removed leaving only the stained membranes (FIG. 13B). Strong lumen-adjacent staining was observable, but also seen under high-power magnification were light spindly cellular membranes that were stained and present throughout the section.

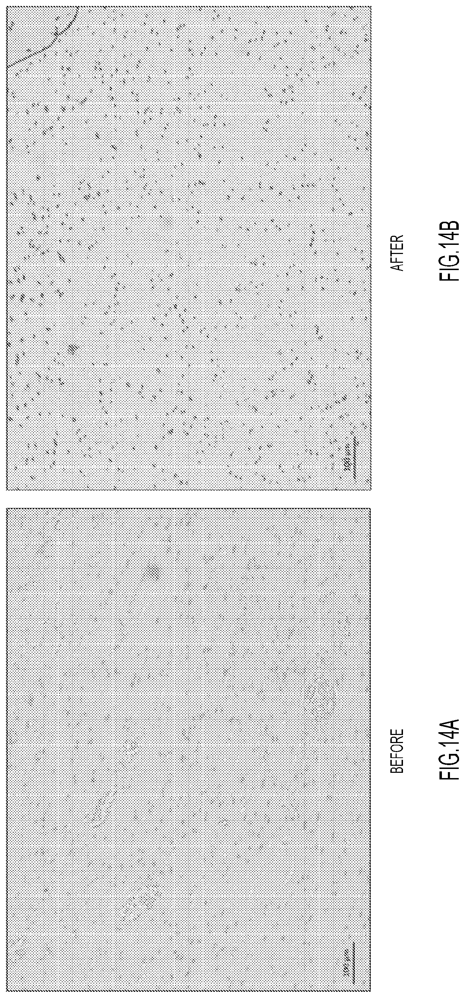

Nuclear procurement was demonstrated in FIGs. 14A and 14B that show colon carcinoma immunostained with an antibody against Ki-67 nuclear protein before and after micropurification. Pink target tumor nuclei were seen on the slide in FIG. 14A (shown as medium gray dots on the grayscale image) and the tumor cell cytoplasm had a light pink blush. After purification, all cytoplasm was removed leaving behind distinctly appearing nuclei (shown as dark gray dots on the grayscale image) on the slide (FIG. 14B).

### Example 8: KRAS gene mutation analysis using HMP

To assess target cell biomolecules after micropurification, three clinical cases of colorectal cancer with a known mutation in the *KRAS* gene were investigated. KRAS has been implicated in a variety of cancers, including colon and breast cancer. After the purification process, DNA in the tumor cells was recovered and subjected to TaqMan PCR analysis to measure mutant *KRAS.*

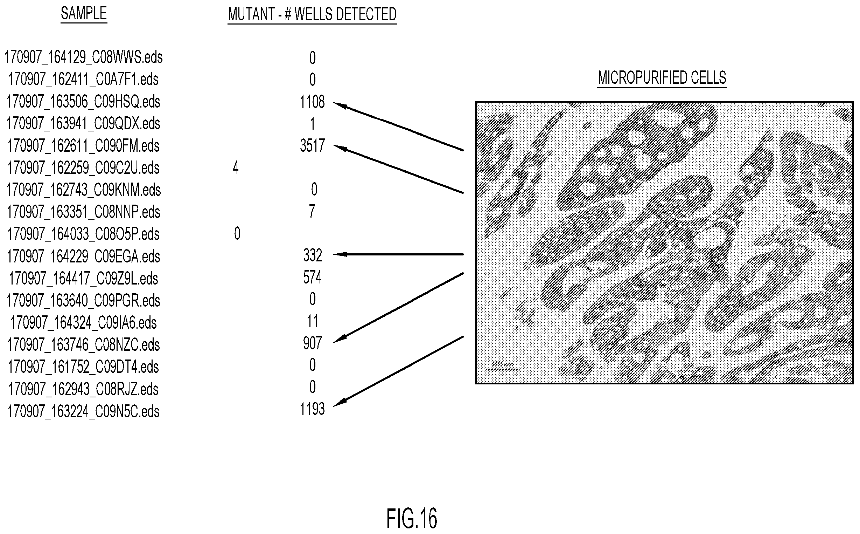

FIG. 16 shows data from a biological sample having a c.34G>A mutation (with corresponding amino acid sequence mutation p.G12S), and also shows that the mutant allele was detected above background in the six tumor containing samples (see arrows), but not in the control samples.

Similar results were observed in the additional two colon cancer cases with a *KRAS* gene mutation (not shown), wherein the mutations were: (a) nucleotide mutation c.35G>T (with corresponding amino acid sequence mutation p.G12V); (b) nucleotide mutation c.34G>A (with corresponding amino acid sequence mutation p.G12D). Overall, 48 micropurification samples (22 mutation positive and 26 controls) were analyzed and mutant KRAS was detected in all of the mutation positive samples but in none of the controls. Thus, the detection sensitivity was 100% and the false positive rate was zero. Accordingly, not only do the HMP assays provided herein allow for DNA recovery, but also, the recovered DNA may be sequenced to identify the presence of mutations.

In general, a variety of different types of gene mutations can be screened for using the techniques presented herein.

Successful detection of a genomic mutation in clinical samples demonstrates that the present techniques and systems are compatible with DNA allelotyping of patient and animal model tissues, as the DNA in the isolated target cells is intact and measurable.

### Example 9: General IHC procedure

A general IHC procedure is provided as follows.
1. Dewax and rehydrate slides by placing in xylenes for 15 minutes (2x) and then in graded alcohols (100%, 95%, 70%) for 2 minutes (in each solution) in the hood.
2. Place slides in diH₂O for 2 minutes (in hood), and make antigen retrieval dilution: 25 ml antigen retrieval solution (10xCitra Plus Buffer "Biogenex") diluted in 225 ml of diH₂O.
3. (Antigen retrieval step) heat slides in diluted 1x Citra plus Buffer "Biogenex" (Step 2) for 5 minutes on 100% power in microwave then add diH₂O to fill tub up to the line (250 ml).
4. (Antigen retrieval step continued) Heat slides in 1x Citra plus Buffer for 15 minutes at 30% power in microwave (pause and add more diH₂O to fill tub up to the line once more).
5. Let slides cool in Citra Plus Buffer for 20 minutes on bench top.
6. Wash slides in new tub of 1x PBS (250 ml).
7. Outline tissue section with hydrophobic pap pen.
8. Apply peroxidase block (Dako or Invitrogen) for 10 minutes and room temperature. (For each slide, apply pap pen outline and immediately add peroxidase block after so as not to let the slide dry. For each slide add 250 µl-300 µl depending on how large the tissue section is to cover the entire section outlined.)
9. After 10 minutes, wash the slide(s) in a fresh tub of 1x PBS (250 ml).
10. Apply primary antibody for 30 minutes at room temperature (RT) (250 µl for each). Decant antibody solution off when finished and proceed to Step 11.
11. Wash slide(s) in a fresh tub of 1x PBS (250 ml).
12. Apply secondary antibody (DAKO Envision+ Anti-Mouse) for 30 minutes at RT (apply 2 to 3 drops to cover slide(s)).
13. Wash slides in fresh 1x PBS (250 ml).
14. Apply DAB solution (prepared and vortexed in a 1.5 mL tube: Dako = 2 drops DAB chromogen + 1 ml substrate buffer) for 5 minutes at RT.
15. Wash slides in diH₂O.
After IHC staining, the slide may be optionally counterstained or may be processed according to the micropurification protocol.

### Example 10: Counterstain procedure

A general counterstain procedure is provided as follows. The slides are placed in each of the following solutions for 30 seconds each (in order).
(a) Hematoxylin
(b) diH₂O
(c) Bluing agent
(d) 70% EtOH
(e) 95% EtOH
(f) 95% EtOH
(g) 100% EtOH

The slides are then placed in each of the following solutions for 2 minutes each (in order):
(h) Xylenes
(i) Xylenes

Thus, the slides are dehydrated through graded alcohol (70%, 95%, 100%) and xylenes for 2 minutes each in the hood. The slides are allowed to air-dry in the hood for at least ten minutes before covering with a coverslip.

### Example 11: Optimized Micropurification Procedure

A general micropurification procedure is provided as follows.
1. Prepare micropurification solution (also referred to as m-pure solution) (In Tris buffer, 0-1.0% concentration of SDS (Sigma Aldrich) and 0.1-1.0 mg/ml proteinase K) and set the heat block to 56° Celsius (C).
2. Wash slides briefly in Tris buffer and place slides one by one on the heating block.
3. Immediately re-circle tissue with pap pen and apply ~250 µl of m-pure solution to each slide. Let sit for 10 minutes (after 5 minutes apply another 250 µl of m-pure solution to each slide).
4. After ten minutes wash in a 0.1% SDS solution in diH₂O (250 ml).
5. Wash in diH₂O and proceed to dewax and cover slip or proceed to DNA, RNA, protein, etc. purification.
It is noted that other suitable micropurification times may be suitable, e.g., ranging from 5 minutes to 30 minutes or any range in between. Additional buffer may be added to prevent slides from drying out.

### Example 12: DNA isolation using micropurified tissue sections

A DNA isolation procedure using a Qiagen GeneRead DNA FFPE Kit is provided as follows.
1. Scrape the slide(s) with a scalpel/razor into a 1.5 ml microcentrifuge tube with 55 µl RNase-free water, 25 µl buffer FTB, and 20 µl proteinase K. Centrifuge so all contents are in the bottom of the tube.
2. Incubate overnight at 56°C in the thermomixer at 400 rpm. (Thermomixer used: Eppendorf Thermomixer C, model # 5382.)
3. Following the overnight step, incubate in the thermomixer at 400 rpm at 90°C for one hour.
4. Briefly centrifuge tubes.
5. Add 115 µl RNase-free water and mix.
6. Add 35 µl UNG (Qiagen), vortex and incubate in thermomixer at 50°C for 1 hour.
7. Briefly centrifuge to remove drops from lid.
8. Add 2 µl RNase A (100 mg/ml) mix, incubate for 2 minutes at RT.
9. Add 250 µl AL Buffer and mix with vortex.
10. Add 250 µl 100% ethanol and mix with vortex.
11. Centrifuge briefly.
12. Transfer 700 µl lysate to qIAamp MinElute column (in a 2 ml collection tube) and centrifuge at max speed for 1 min.
13. Discard flow-through and reuse collection tube. Repeat step 12 until all lysate is used.
14. Add 500 µl AW1 Buffer to spin column and centrifuge at max speed for 1 min. Discard flow through and reuse collection tube.
15. Add 500 µl AW2 Buffer to spin column and centrifuge at max speed for 1 min. Discard flow through and reuse collection tube.
16. Add 250 µl 100 % ethanol to spin column and centrifuge at max speed for 1 min. Discard flow-through and collection tube.
17. Place spin column in new 2 ml collection tube and centrifuge at max speed for 1 min.
18. Discard the collection tube and place in newly labeled 1.5 ml microcentrifuge tube and add 30 µl ATE Buffer to the column.
19. Incubate at RT for 5 min and centrifuge at max speed for 1 min. Discard MinElute spin column and keep eluted DNA for further analysis. Store at -20°C for long term storage.

### Example 13. RNA isolation from micropurified tissue sections

An RNA isolation procedure based on Qiagen's RNeasy FFPE Tissue Section kit is provided as follows.
1. Scrape slide(s) with a scalpel/ razor into a 1.5 ml microcentrifuge tube with 150 µl PKD buffer.
2. Add 10 µl proteinase K. Mix gently by pipetting up and down.
3. Incubate at 56°C for 15 min, then at 80°C for 15 min.
4. Incubate on ice for 3 min. Then, centrifuge for 15 min at 20,000 x g (13,500 rpm).
5. Transfer the supernatant to a new microcentrifuge tube taking care not to disturb the pellet.
6. Add DNase Booster Buffer equivalent to a tenth of the total sample volume (approximately 16 µl) and 10 µl DNase 1 stock solution. Mix by inverting the tube. Centrifuge briefly to collect residual liquid from the sides of the tube.
7. Incubate at RT for 15 min.
8. Add 720 µl ethanol (100%) to the sample, and mix well by pipetting. Do not centrifuge. Proceed immediately to step 9.
9. Transfer 700 µl of the sample, including any precipitates that may have formed, to a RNeasy MinElute spin column placed in a 2 ml collection tube. Close the lid gently, and centrifuge for 15 seconds at greater than or equal to 8000 x g (greater than or equal to 10,000 rpm). Discard the flow through.
10. Repeat step 9 until the entire sample has passed through the RNeasy MinElute spin column.
11. Add 500 µl RPE Buffer to the RNeasy MinElute spin column. Close the lid gently, and centrifuge for 15 seconds at greater than or equal to 8000 x g (greater than or equal to 10,000 rpm) to wash the spin column membrane. Discard the flow through.
12. Add 500 µl RPE Buffer to the RNeasy MinElute spin column. Close the lid gently, and centrifuge for 2 min at greater than or equal to 8000 x g (greater than or equal to 10,000 rpm) to wash the spin column membrane. Discard the collection tube with the flow through.
13. Place the RNeasy MinElute spin column in a new 2 ml collection tube (supplied). Open the lid of the spin column, and centrifuge at full speed for 5 min. Discard the collection tube with the flow through.
14. Place the RNeasy MinElute spin column in a new 1.5 ml collection tube. Add 14-30 µl (we use 30 µl) RNase-free water directly to the spin column membrane. Close the lid gently, and centrifuge for 1 min at full speed to elute the RNA.

### Example 14. Isolation of nuclei with NBT/BCIP using micropurification

Nuclei from human breast cancer FFPE tissue was successfully isolated via micropurification using a histone primary antibody and a secondary antibody labeled with alkaline phosphatase. The chromogen was NBT/BCIP. The standard IHC staining procedure described herein was used. For micropurification, a 1mg/mL proteinase K and 1% SDS solution was contacted with the sample for about 10 minutes at 56°C. Corresponding images are shown in FIGs. 19A-19D.

### REFERENCES

[1] Brower V: NCI-MATCH pairs tumor mutations with matching drugs. Nature biotechnology 2015, 33:790-1.
[2] Cancer Genome Atlas N: Comprehensive molecular portraits of human breast tumours. Nature 2012, 490:61-70.
[3] Cancer Genome Atlas Research N: Comprehensive genomic characterization of squamous cell lung cancers. Nature 2012, 489:519-25.
[4] Cecchi F, Lih CJ, Lee YH, Walsh W, Rabe DC, Williams PM, Bottaro DP: Expression array analysis of the hepatocyte growth factor invasive program. Clinical & experimental metastasis 2015, 32:659-76.
[5] Frampton GM, Fichtenholtz A, Otto GA, Wang K, Downing SR, He J, Schnall-Levin M, White J, Sanford EM, An P, Sun J, Juhn F, Brennan K, Iwanik K, Maillet A, Buell J, White E, Zhao M, Balasubramanian S, Terzic S, Richards T, Banning V, Garcia L, Mahoney K, Zwirko Z, Donahue A, Beltran H, Mosquera JM, Rubin MA, Dogan S, Hedvat CV, Berger MF, Pusztai L, Lechner M, Boshoff C, Jarosz M, Vietz C, Parker A, Miller VA, Ross JS, Curran J, Cronin MT, Stephens PJ, Lipson D, Yelensky R: Development and validation of a clinical cancer genomic profiling test based on massively parallel DNA sequencing. Nature biotechnology 2013, 31:1023-31.
[6] Fumagalli D, Gavin PG, Taniyama Y, Kim SI, Choi HJ, Paik S, Pogue-Geile KL: A rapid, sensitive, reproducible and cost-effective method for mutation profiling of colon cancer and metastatic lymph nodes. BMC cancer 2010, 10:101.
[7] Garcia-Murillas I, Lambros M, Turner NC: Determination of HER2 amplification status on tumour DNA by digital PCR. PloS one 2013, 8:e83409.
[8] Grosserueschkamp F, Bracht T, Diehl HC, Kuepper C, Ahrens M, Kallenbach-Thieltges A, Mosig A, Eisenacher M, Marcus K, Behrens T, Bruning T, Theegarten D, Sitek B, Gerwert K: Spatial and molecular resolution of diffuse malignant mesothelioma heterogeneity by integrating label-free FTIR imaging, laser capture microdissection and proteomics. Scientific reports 2017, 7:44829.
[9] Kummar S, Williams PM, Lih CJ, Polley EC, Chen AP, Rubinstein LV, Zhao Y, Simon RM, Conley BA, Doroshow JH: Application of molecular profiling in clinical trials for advanced metastatic cancers. J Natl Cancer Inst 2015, 107.
[10] Lih CJ, Sims DJ, Harrington RD, Polley EC, Zhao Y, Mehaffey MG, Forbes TD, Das B, Walsh WD, Datta V, Harper KN, Bouk CH, Rubinstein LV, Simon RM, Conley BA, Chen AP, Kummar S, Doroshow JH, Williams PM: Analytical Validation and Application of a Targeted Next-Generation Sequencing Mutation-Detection Assay for Use in Treatment Assignment in the NCI-MPACT Trial. J Mol Diagn 2015.
[11] Linton K, Howarth C, Wappett M, Newton G, Lachel C, Iqbal J, Pepper S, Byers R, Chan WJ, Radford J: Microarray gene expression analysis of fixed archival tissue permits molecular classification and identification of potential therapeutic targets in diffuse large B-cell lymphoma. J Mol Diagn 2012, 14:223-32.
[12] Nielsen T, Wallden B, Schaper C, Ferree S, Liu S, Gao D, Barry G, Dowidar N, Maysuria M, Storhoff J: Analytical validation of the PAM50-based Prosigna Breast Cancer Prognostic Gene Signature Assay and nCounter Analysis System using formalin-fixed paraffin-embedded breast tumor specimens. BMC cancer 2014, 14:177.
[13] Scott DW, Wright GW, Williams PM, Lih CJ, Walsh W, Jaffe ES, Rosenwald A, Campo E, Chan WC, Connors JM, Smeland EB, Mottok A, Braziel RM, Ott G, Delabie J, Tubbs RR, Cook JR, Weisenburger DD, Greiner TC, Glinsmann-Gibson BJ, Fu K, Staudt LM, Gascoyne RD, Rimsza LM: Determining cellof-origin subtypes of diffuse large B-cell lymphoma using gene expression in formalin-fixed paraffin embedded tissue. Blood 2014, 123:1214-7.
[14] Sparano JA, Gray RJ, Makower DF, Pritchard KI, Albain KS, Hayes DF, Geyer CE, Jr., Dees EC, Perez EA, Olson JA, Jr., Zujewski J, Lively T, Badve SS, Saphner TJ, Wagner LI, Whelan TJ, Ellis MJ, Paik S, Wood WC, Ravdin P, Keane MM, Gomez Moreno HL, Reddy PS, Goggins TF, Mayer IA, Brufsky AM, Toppmeyer DL, Kaklamani VG, Atkins JN, Berenberg JL, Sledge GW: Prospective Validation of a 21-Gene Expression Assay in Breast Cancer. The New England journal of medicine 2015, 373:2005-14.
[15] Yan W, Shih JH, Rodriguez-Canales J, Tangrea MA, Ylaya K, Hipp J, Player A, Hu N, Goldstein AM, Taylor PR, Emmert-Buck MR, Erickson HS: Identification of unique expression signatures and therapeutic targets in esophageal squamous cell carcinoma. BMC Res Notes 2012, 5:73.
[16] Zhuang Z, Bertheau P, Emmert-Buck MR, Liotta LA, Gnarra J, Linehan WM, Lubensky IA: A microdissection technique for archival DNA analysis of specific cell populations in lesions < 1 mm in size. The American journal of pathology 1995, 146:620-5.
[17] Bonner RF, Emmert-Buck M, Cole K, Pohida T, Chuaqui R, Goldstein S, Liotta LA: Laser capture microdissection: molecular analysis of tissue. Science 1997, 278:1481,3.
[18] Eberle FC, Hanson JC, Killian JK, Wei L, Ylaya K, Hewitt SM, Jaffe ES, Emmert-Buck MR, Rodriguez-Canales J: Immunoguided laser assisted microdissection techniques for DNA methylation analysis of archival tissue specimens. J Mol Diagn 2010, 12:394-401.
[19] Emmert-Buck MR, Bonner RF, Smith PD, Chuaqui RF, Zhuang Z, Goldstein SR, Weiss RA, Liotta LA: Laser capture microdissection. Science 1996, 274:998-1001.
[20] Emmert-Buck MR, Roth MJ, Zhuang Z, Campo E, Rozhin J, Sloane BF, Liotta LA, Stetler-Stevenson WG: Increased gelatinase A (MMP-2) and cathepsin B activity in invasive tumor regions of human colon cancer samples. The American journal of pathology 1994, 145:1285-90.
[21] Fend F, Emmert-Buck MR, Chuaqui R, Cole K, Lee J, Liotta LA, Raffeld M: Immuno-LCM: laser capture microdissection of immunostained frozen sections for mRNA analysis. The American journal of pathology 1999, 154:61-6.
[22] Hipp J, Cheng J, Hanson JC, Yan W, Taylor P, Hu N, Rodriguez-Canales J, Tangrea MA, Emmert-Buck MR, Balis U: SIVQ-aided laser capture microdissection: A tool for high-throughput expression profiling. J Pathol Inform 2011, 2:19.
[23] Simone NL, Remaley AT, Charboneau L, Petricoin EF, 3rd, Glickman JW, Emmert-Buck MR, Fleisher TA, Liotta LA: Sensitive immunoassay of tissue cell proteins procured by laser capture microdissection. The American journal of pathology 2000, 156:445-52.
[24] Tangrea MA, Chuaqui RF, Gillespie JW, Ahram M, Gannot G, Wallis BS, Best CJ, Linehan WM, Liotta LA, Pohida TJ, Bonner RF, Emmert-Buck MR: Expression microdissection: operator independent retrieval of cells for molecular profiling. Diagn Mol Pathol 2004, 13:207-12.
[25] Tangrea MA, Hanson JC, Bonner RF, Pohida TJ, Rodriguez-Canales J, Emmert-Buck MR: Immunoguided microdissection techniques. Methods Mol Biol 2011, 755:57-66.
[26] Fearon ER, Hamilton SR, Vogelstein B: Clonal analysis of human colorectal tumors. Science 1987, 238:193-7.
[27] Radford DM, Fair K, Thompson AM, Ritter JH, Holt M, Steinbrueck T, Wallace M, Wells SA, Jr., Donis-Keller HR: Allelic loss on a chromosome 17 in ductal carcinoma in situ of the breast. Cancer research 1993, 53:2947-9.
[28] Shibata D, Hawes D, Li ZH, Hernandez AM, Spruck CH, Nichols PW: Specific genetic analysis of microscopic tissue after selective ultraviolet radiation fractionation and the polymerase chain reaction. The American journal of pathology 1992, 141:539-43.
[29] Kovach JS, McGovern RM, Cassady JD, Swanson SK, Wold LE, Vogelstein B, Sommer SS: Direct sequencing from touch preparations of human carcinomas: analysis of p53 mutations in breast carcinomas. J Natl Cancer Inst 1991, 83:1004-9.
[30] Noguchi S, Motomura K, Inaji H, Imaoka S, Koyama H: Clonal analysis of predominantly intraductal carcinoma and precancerous lesions of the breast by means of polymerase chain reaction. Cancer research 1994, 54:1849-53.
[31] Park TW, Felix JC, Wright TC, Jr.: X chromosome inactivation and microsatellite instability in early and advanced bilateral ovarian carcinomas. Cancer research 1995, 55:4793-6.
[32] Chuaqui RF, Sanz-Ortega J, Vocke C, Linehan WM, Sanz-Esponera J, Zhuang Z, Emmert-Buck MR, Merino MJ: Loss of heterozygosity on the short arm of chromosome 8 in male breast carcinomas. Cancer research 1995, 55:4995-8.
[33] Best CJ, Gillespie JW, Yi Y, Chandramouli GV, Perlmutter MA, Gathright Y, Erickson HS, Georgevich L, Tangrea MA, Duray PH, Gonzalez S, Velasco A, Linehan WM, Matusik RJ, Price DK, Figg WD, Emmert-Buck MR, Chuaqui RF: Molecular alterations in primary prostate cancer after androgen ablation therapy. Clin Cancer Res 2005, 11:6823-34.
[34] Chowdhuri SR, Xi L, Pham TH, Hanson J, Rodriguez-Canales J, Berman A, Rajan A, Giaccone G, Emmert-Buck M, Raffeld M, Filie AC: EGFR and KRAS mutation analysis in cytologic samples of lung adenocarcinoma enabled by laser capture microdissection. Mod Pathol 2011, 25:548-55.
[35] Chuaqui RF, Bonner RF, Best CJ, Gillespie JW, Flaig MJ, Hewitt SM, Phillips JL, Krizman DB, Tangrea MA, Ahram M, Linehan WM, Knezevic V, Emmert-Buck MR: Post-analysis follow-up and validation of microarray experiments. Nat Genet 2002, 32 Suppl:509-14.
[36] Johann DJ, Rodriguez-Canales J, Mukherjee S, Prieto DA, Hanson JC, Emmert-Buck M, Blonder J: Approaching solid tumor heterogeneity on a cellular basis by tissue proteomics using laser capture microdissection and biological mass spectrometry. J Proteome Res 2009, 8:2310-8.
[37] Jumper N, Hodgkinson T, Paus R, Bayat A: Site-specific gene expression profiling as a novel strategy for unravelling keloid disease pathobiology. PloS one 2017, 12:e0172955.
[38] Cubi R, Vembar SS, Biton A, Franetich JF, Bordessoulles M, Sossau D, Zanghi G, Bosson-Vanga H, Benard M, Moreno A, Dereuddre-Bosquet N, Le Grand R, Scherf A: Laser capture microdissection enables transcriptomic analysis of dividing and quiescent liver stages of Plasmodium relapsing species. 2017.
[39] Ebrahimi A, Jung MH, Dreyfuss JM, Pan H, Sgroi D, Bonner-Weir S, Weir GC: Evidence of stress in beta cells obtained with laser capture microdissection from pancreases of brain dead donors. Islets 2017, 9:19-29.
[40] Serova TA, Tikhonovich IA, Tsyganov VE: Analysis of nodule senescence in pea (Pisum sativum L.) using laser microdissection, real-time PCR, and ACC immunolocalization. Journal of plant physiology 2017, 212:29-44.
[41] Adebayo Michael AO, Ahsan N, Zabala V, Francois-Vaughan H, Post S, Brilliant KE, Salomon AR, Sanders JA, Gruppuso PA: Proteomic analysis of laser capture microdissected focal lesions in a rat model of progenitor marker-positive hepatocellular carcinoma. Oncotarget 2017.
[42] Costa S, Correia-de-Sa P, Porto MJ, Caine L: The Use of Laser Microdissection in Forensic Sexual Assault Casework: Pros and Cons Compared to Standard Methods. Journal of forensic sciences 2017.
[43] Ning X, Yin Z, Li Z, Xu J, Wang L, Shen W, Lu Y, Cai G, Zhang X, Chen X: Comparative proteomic analysis of urine and laser microdissected glomeruli in IgA nephropathy. Clinical and experimental pharmacology & physiology 2017.
[44] Hobeika L, Barati MT, Caster DJ, McLeish KR, Merchant ML: Characterization of glomerular extracellular matrix by proteomic analysis of laser-captured microdissected glomeruli. Kidney international 2017, 91:501-11.
[45] Mollee P, Boros S, Loo D, Ruelcke JE, Lakis VA, Cao KL, Renaut P, Hill MM: Implementation and evaluation of amyloidosis subtyping by laser-capture microdissection and tandem mass spectrometry. Clinical proteomics 2016, 13:30.
[46] Zhang L, Lanzoni G, Battarra M, Inverardi L, Zhang Q: Proteomic profiling of human islets collected from frozen pancreata using laser capture microdissection. Journal of proteomics 2017, 150:149-59.
[47] Gillespie JW, Gannot G, Tangrea MA, Ahram M, Best CJ, Bichsel VE, Petricoin EF, Emmert-Buck MR, Chuaqui RF: Molecular profiling of cancer. Toxicol Pathol 2004, 32 Suppl 1:67-71.
[48] Erickson HS, Gillespie JW, Emmert-Buck MR: Tissue microdissection. Methods Mol Biol 2008, 424:433-48.
[49] Bova GS, Eltoum IA, Kiernan JA, Siegal GP, Frost AR, Best CJ, Gillespie JW, Su GH, Emmert-Buck MR: Optimal molecular profiling of tissue and tissue components: defining the best processing and microdissection methods for biomedical applications. Mol Biotechnol 2005, 29:119-52.
[50] Rosenberg AZ, Armani MD, Fetsch PA, Xi L, Pham TT, Raffeld M, Chen Y, O'Flaherty N, Stussman R, Blackler AR, Du Q, Hanson JC, Roth MJ, Filie AC, Roh MH, Emmert-Buck MR, Hipp JD, Tangrea MA: High-Throughput Microdissection for Next-Generation Sequencing. PloS one 2016, 11:e0151775.
[51] Quiros PM, Goyal A, Jha P, Auwerx J: Analysis of mtDNA/nDNA Ratio in Mice. Current protocols in mouse biology 2017, 7:47-54.
[52] Evdokimovskii EV, Gubina NE, Ushakova TE, Gaziev AI: [Changes of mitochondrial DNA/nuclear DNA ratio in the blood serum following X-ray irradiation of mice at various doses]. Radiatsionnaia biologiia, radioecologiia 2012, 52:565-71.
[53] Kampira E, Dzobo K, Kumwenda J, van Oosterhout JJ, Parker MI, Dandara C: Peripheral blood mitochondrial DNA/nuclear DNA (mtDNA/nDNA) ratio as a marker of mitochondrial toxicities of stavudine containing antiretroviral therapy in HIV-infected Malawian patients. Omics: a journal of integrative biology 2014, 18:438-45.
[54] Allio R, Donega S, Galtier N, Nabholz B: Large Variation in the Ratio of Mitochondria) to Nuclear Mutation Rate across Animals: Implications for Genetic Diversity and the Use of Mitochondria) DNA as a Molecular Marker. Molecular biology and evolution 2017, 34:2762-72.

## Claims

1. A method of collecting cells comprising:
contacting a histological sample affixed to a surface with a specific probe to form a bound complex comprising cells labeled with said specific probe, wherein said specific probe comprises a reactive moiety;
contacting said bound complex comprising the reactive moiety with a substrate that reacts with said reactive moiety to deposit a chemical material onto the surface of said cells labeled with said specific probe, wherein said chemical material forms a barrier covering the labeled cells or subcellular structures; and
applying a micropurification solution to the biological sample, wherein said micropurification solution degrades, digests, or otherwise differentially processes cells or structures not protected by the barrier; wherein:
(i) said specific probe comprises an antibody, a nucleotide-based hybridization probe, an affimer, or an aptamer;
(ii) said reactive moiety comprises an enzyme, with said substrate being a substrate for said enzyme; and
(iii) said substrate is a chromogenic reagent that reacts with the reactive moiety to produce an insoluble chemical material.

2. The method of claim 1, wherein a plurality of specific probes is used, and wherein each probe binds to a different target.

3. The method of claim 1 or 2, wherein said antibody comprises a primary antibody and a secondary antibody, wherein said primary antibody specifically binds specific cells or cellular structures in said tissue, and wherein said secondary antibody specifically binds said primary antibody and comprises an enzyme as reactive moiety capable of reacting with said substrate to form said barrier.

4. The method of claim 3, wherein said antibody binds to a tumor marker, a cancer marker, a nuclear marker, an extracellular marker, an intracellular marker, or an immunological marker.

5. The method of claim 1, further comprising collecting cells covered by the barrier, wherein the cells collected are selected from the group consisting of neoplastic cells, cancer cells, tumor cells, immune cells, normal cells, subcellular compartments, and structural components of a tissue.

6. The method of any one of claims 1-5, further comprising collecting cells that are not covered by the barrier.

7. The method of claim 1 or 2, wherein said nucleotide-based hybridization probe is complementary to a DNA sequence comprising a mutation.

8. The method of any one of claims 1-7, wherein said enzyme comprised in said reactive moiety is horseradish peroxidase or alkaline phosphatase.

9. The method of any one of claims 1-8, wherein the chromogenic reagent is selected from the group consisting of 3-amino-9-ethylcarbazole (AEC), 3,3'-diaminobenzidine (DAB) with or without nickel enhancer, tetramethyl benzidine (TMB), 5-bromo-4-chloro-3indolyl-phosphate/nitro blue tetrazolium (BCIP/NBT), naphthol AS-MX phosphate + Fast Blue BB, naphthol AS-MX phosphate + Fast Red TR, naphthol AS-MX phosphate + new fuchsin; and nitro blue tetrazolium (NBT).

10. The method of claim 8 or 9, wherein said enzyme is horseradish peroxidase and said chromogenic reagent is 3,3'-diaminobenzidine (DAB).

11. The method of claim 8 or 9, wherein said enzyme is alkaline phosphatase and said chromogenic reagent is Fast Red TR.

12. The method of claim 5, further comprising:
isolating DNA from the collected cells and determining a DNA allelotype; or
analyzing the collected cells using a method comprising one or more of next generation sequencing (NGS), expression profiling, proteomic analysis, lipid analysis, metabolic analysis, and/or immunological activity analysis.

13. The method of any one of claims 1-12, wherein the cells are embedded in paraffin.

14. The method of claim 1, wherein said sample is formalin-fixed.

## Patentansprüche

1. Verfahren zum Sammeln von Zellen, umfassend:
Inkontaktbringen einer histologischen Probe, die an einer Oberfläche angebracht ist, mit einer spezifischen Sonde, um einen gebundenen Komplex zu bilden, der die mit der spezifischen Sonde markierten Zellen umfasst, wobei die spezifische Sonde eine reaktive Einheit aufweist;
Inkontaktbringen des gebundenen Komplexes, der die reaktive Einheit aufweist, mit einem Substrat, das mit der reaktiven Einheit reagiert, um ein chemisches Material auf der Oberfläche der mit der spezifischen Sonde markierten Zellen abzuscheiden, wobei das chemische Material eine Barriere formt, welche die markierten Zellen oder subzellulären Strukturen bedeckt; und
Applizieren einer Mikropurifikationslösung auf die biologische Probe, wobei die Mikropurifikationslösung die Zellen oder Strukturen, die von der Barriere nicht geschützt sind, abbaut, aufschließt oder anderweitig differentiell prozessiert; wobei:
(i) die spezifische Sonde einen Antikörper, eine nukleotid-basierte Hybridisierungssonde, ein Affimer oder ein Aptamer umfasst;
(ii) die reaktive Einheit ein Enzym umfasst, wobei das Substrat ein Substrat für das Enzym ist; und
(iii) das Substrat ein chromogenes Reagenz ist, das mit der reaktiven Einheit reagiert, um ein unlösliches chemisches Material zu bilden.

2. Verfahren gemäß Anspruch 1, wobei eine Mehrzahl an spezifischen Sonden verwendet wird, und wobei jede Sonde an ein unterschiedliches Target bindet.

3. Verfahren gemäß Anspruch 1 oder 2, wobei der Antikörper einen primären Antikörper und einen sekundären Antikörper umfasst, wobei der primäre Antikörper spezifisch an spezifische Zellen oder zelluläre Strukturen in dem Gewebe bindet, und wobei der sekundäre Antikörper spezifisch an den primären Antikörper bindet und ein Enzym als reaktive Einheit aufweist, welches geeignet ist, mit dem Substrat zu reagieren, um die Barriere zu bilden.

4. Verfahren gemäß Anspruch 3, wobei der Antikörper an einen Tumormarker, einen Krebsmarker, einen nukleären Marker, einen extrazellulären Marker, einen intrazellulären Marker oder einen immunologischen Marker bindet.

5. Verfahren gemäß Anspruch 1, ferner umfassend das Sammeln der Zellen, die von der Barriere bedeckt sind, wobei die gesammelten Zellen aus der Gruppe ausgewählt sind, die aus neoplastischen Zellen, Krebszellen, Tumorzellen, Immunzellen, normalen Zellen, subzellulären Kompartimenten und strukturellen Komponenten eines Gewebes besteht.

6. Verfahren gemäß einem der Ansprüche 1-5, ferner umfassend das Sammeln der Zellen, die nicht von der Barriere bedeckt sind.

7. Verfahren gemäß Anspruch 1 oder 2, wobei die nukleotid-basierte Hybridisierungssonde komplementär zu einer DNA-Sequenz ist, die eine Mutation aufweist.

8. Verfahren gemäß einem der Ansprüche 1-7, wobei das in der reaktiven Einheit umfasste Enzym Meerrettichperoxidase oder alkalische Phosphatase ist.

9. Verfahren gemäß einem der Ansprüche 1-8, wobei das chromogene Reagenz aus der Gruppe ausgewählt wird, die aus 3-Amino-9-ethylcarbazol (AEC), 3,3'-Diaminobenzidin (DAB) mit oder ohne Nickel-Enhancer, Tetramethylbenzidin (TMB), 5-Brom-4-chlor-3-indolyl-phosphat/Nitroblautetrazolium (BCIP/NBT), Naphthol AS-MX Phosphat + Fast Blue BB, Naphthol AS-MX Phosphat + Fast Red TR, Naphthol AS-MX Phosphat + Neufuchsin; und Nitroblautetrazolium (NBT) besteht.

10. Verfahren gemäß Anspruch 8 oder 9, wobei das Enzym Meerrettichperoxidase und das chromogene Reagenz 3,3'-Diaminobenzidin (DAB) ist.

11. Verfahren gemäß Anspruch 8 oder 9, wobei das Enzym alkalische Phosphatase und das chromogene Reagenz Fast Red TR ist.

12. Verfahren gemäß Anspruch 5, ferner umfassend:
Isolieren von DNA aus den gesammelten Zellen und Bestimmen eines DNA-Alleltyps; oder
Analysieren der gesammelten Zellen unter Verwendung eines Verfahrens, das eines oder mehr von Next Generation Sequencing (NGS), Expressionsanalyse, Proteomanalyse, Lipidanalyse, Stoffwechselanalyse und/oder immunologischer Aktivitätsanalyse umfasst.

13. Verfahren gemäß einem der Ansprüche 1-12, wobei die Zellen in Paraffin eingebettet sind.

14. Verfahren gemäß Anspruch 1, wobei die Probe mit Formalin fixiert ist.

## Revendications

1. Procédé de prélèvement de cellules comprenant:
la mise en contact d'un échantillon histologique fixé sur une surface avec une sonde spécifique afin de former un complexe lié comprenant des cellules marquées par ladite sonde spécifique, ladite sonde spécifique comprenant une fraction réactive ;
la mise en contact dudit complexe lié comprenant la fraction réactive avec un substrat qui réagit avec ladite fraction réactive afin de déposer un matériau chimique sur la surface desdites cellules marquées par ladite sonde spécifique, ledit matériau chimique formant une barrière recouvrant les cellules ou structures subcellulaires marquées; et
appliquer d'une solution de micropurification à l'échantillon biologique, ladite solution de micropurification dégradant, digérant ou traitant différemment les cellules ou structures non protégées par la barrière ; dans lequel :
(i) ladite sonde spécifique comprend un anticorps, une sonde d'hybridation nucléotidique, un affimère ou un aptamère ;
(ii) ladite fraction réactive comprend une enzyme, ledit substrat étant un substrat pour ladite enzyme ; et
(iii) ledit substrat est un réactif chromogène qui réagit avec la fraction réactive pour produire une substance chimique insoluble.

2. Procédé selon la revendication 1, dans lequel plusieurs sondes spécifiques sont utilisées, chaque sonde se liant à une cible différente.

3. Procédé selon la revendication 1 ou 2, dans lequel ledit anticorps comprend un anticorps primaire et un anticorps secondaire, ledit anticorps primaire se liant spécifiquement à des cellules ou structures cellulaires spécifiques dudit tissu, et ledit anticorps secondaire se liant spécifiquement audit anticorps primaire et comprenant une enzyme comme fraction réactive capable de réagir avec ledit substrat pour former ladite barrière.

4. Procédé selon la revendication 3, dans lequel ledit anticorps se lie à un marqueur tumoral, un marqueur du cancer, un marqueur nucléaire, un marqueur extracellulaire, un marqueur intracellulaire ou un marqueur immunologique.

5. Procédé selon la revendication 1, comprenant en outre la collecte de cellules recouvertes par la barrière, les cellules collectées étant choisies parmi les cellules néoplasiques, cancéreuses, tumorales, immunitaires, normales, les compartiments subcellulaires et les composants structurels d'un tissu.

6. Procédé selon l'une quelconque des revendications 1 à 5, comprenant en outre la collecte de cellules non recouvertes par la barrière.

7. Procédé selon la revendication 1 ou 2, dans lequel ladite sonde d'hybridation nucléotidique est complémentaire d'une séquence d'ADN comprenant une mutation.

8. Procédé selon l'une quelconque des revendications 1 à 7, dans lequel ladite enzyme comprise dans ledit fragment réactif est la peroxydase de raifort ou la phosphatase alcaline.

9. Procédé selon l'une quelconque des revendications 1 à 8, dans lequel le réactif chromogène est choisi dans le groupe constitué par le 3-amino-9-éthylcarbazole (AEC), la 3,3'-diaminobenzidine (DAB) avec ou sans activateur de nickel, la tétraméthylbenzidine (TMB), le 5-bromo-4-chloro-3-indolyl-phosphate/nitro bleu tétrazolium (BCIP/NBT), le naphtol AS-MX phosphate + Fast Blue BB, le naphtol AS-MX phosphate + Fast Red TR, le naphtol AS-MX phosphate + nouvelle fuchsine; et le nitro bleu tétrazolium (NBT).

10. Procédé selon la revendication 8 ou 9, dans lequel ladite enzyme est la peroxydase de raifort et ledit réactif chromogène est la 3,3'-diaminobenzidine (DAB).

11. Procédé selon la revendication 8 ou 9, dans lequel ladite enzyme est la phosphatase alcaline et ledit réactif chromogène est le Fast Red TR.

12. Procédé selon la revendication 5, comprenant en outre:
isoler de l'ADN des cellules collectées et la détermination d'un allélotype d'ADN; ou
analyser des cellules collectées par une méthode comprenant une ou plusieurs méthodes parmi le séquençage de nouvelle génération (NGS), le profilage d'expression, l'analyse protéomique, l'analyse lipidique, l'analyse métabolique et/ou l'analyse de l'activité immunologique.

13. Procédé selon l'une quelconque des revendications 1 à 12, dans lequel les cellules sont incluses dans de la paraffine.

14. Procédé selon la revendication 1, dans lequel ledit échantillon est fixé au formol.
